(19) Europäisches Patentamt
European Patent Office
Office européen des brevets

(11) EP 2 899 272 B1

(12) EUROPEAN PATENT SPECIFICATION

(45) Date of publication and mention of the grant of the patent:
**15.08.2018 Bulletin 2018/33**

(21) Application number: **13838373.2**

(22) Date of filing: **19.09.2013**

(51) Int Cl.:
***C12Q 1/6886*** *(2018.01)*

(86) International application number:
**PCT/JP2013/075334**

(87) International publication number:
**WO 2014/046197 (27.03.2014 Gazette 2014/13)**

(54) **METHOD AND USE FOR OBTAINING INFORMATION ABOUT COLORECTAL CANCER**

VERFAHREN UND VERWENDUNG ZUR GEWINNUNG VON INFORMATIONEN ÜBER KOLOREKTALKREBS

MÉTHODE ET UTILISATION PERMETTANT D'OBTENIR DES INFORMATIONS SUR LE CANCER COLORECTAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.09.2012 JP 2012205762**
**29.05.2013 JP 2013113436**

(43) Date of publication of application:
**29.07.2015 Bulletin 2015/31**

(73) Proprietors:
- **Sysmex Corporation**
  **Kobe-shi, Hyogo 651-0073 (JP)**
- **The University of Tokyo**
  **Tokyo 113-8654 (JP)**

(72) Inventors:
- **SAKAI, Ayako**
  **Kobe-shi**
  **Hyogo 651-0073 (JP)**
- **NAGAE, Genta**
  **Tokyo 113-8654 (JP)**
- **MIDORIKAWA, Yutaka**
  **Tokyo 113-8654 (JP)**
- **KAJITA, Masahiro**
  **Gunma 3700033 (JP)**
- **ABURATANI, Hiroyuki**
  **Tokyo 1138-654 (JP)**

(74) Representative: **Paemen, Liesbet R.J. et al**
**De Clercq & Partners**
**Edgard Gevaertdreef 10 a**
**9830 Sint-Martens-Latem (BE)**

(56) References cited:
**WO-A1-2011/002029**
**WO-A1-2012/046871**
**JP-A- 2009 539 404**
**JP-A- 2012 506 253**
**US-A1- 2012 196 756**

- **YOUNG-HO KIM ET AL: "Epigenomic Analysis of Aberrantly Methylated Genes in Colorectal Cancer Identifies Genes Commonly Affected by Epigenetic Alterations", ANNALS OF SURGICAL ONCOLOGY, SPRINGER-VERLAG, NE, vol. 18, no. 8, 5 February 2011 (2011-02-05), pages 2338-2347, XP019926925, ISSN: 1534-4681, DOI: 10.1245/S10434-011-1573-Y**
- **MYOUNG SOOK KIM ET AL: "DNA methylation markers in colorectal cancer", CANCER AND METASTASIS REVIEWS, KLUWER ACADEMIC PUBLISHERS, DO, vol. 29, no. 1, 6 February 2010 (2010-02-06), pages 181-206, XP019787671, ISSN: 1573-7233**
- **HIROSHI HAYASHI ET AL.: 'Genome Tiling Array o Mochiita Morateki Methyl-ka Kenshutsuho no Kaihatsu' DAI 64 KAI PROCEEDINGS OF THE JAPANESE CANCER ASSOCIATION 2005, page 112, XP008178704**
- **HIROSHI HAYASHI ET AL.: 'Genome Tiling Array ni yoru Komitsudo Methyl-ka Kaiseki' THE JAPAN SOCIETY OF HUMAN GENETICS DAI 52 KAI TAIKAI PROGRAMOSHOROKUSHU 2007, page 176, XP008178679**
- **KIBRIYA,M.G. ET AL.: 'A genome-wide DNA methylation study in colorectal carcinoma.' BMC MED.GENOMICS vol. 4, no. 50, 2011, pages 1 - 16, XP021104259**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **FANG,W.-J. ET AL.: 'Genome-wide Analysis of Aberrant DNA Methylation for Identification of Potential Biomarkers in Colorectal Cancer Patients.' ASIAN PACIFIC JOURNAL OF CANCER PREVENTION vol. 13, no. 5, May 2012, pages 1917 - 1921, XP055242532**
- **SHUICHI TSUTSUMI ET AL.: 'Komitsudo Tiling Array o Mochiita chIP-chip Kaiseki' DAI 65 KAI PROCEEDINGS OF THE JAPANESE CANCER ASSOCIATION 2006, page 169, XP008178706**


Remarks:

The file contains technical information submitted after the application was filed and not included in this specification

**Description**

## TECHNICAL FIELD

The present invention relates to a method for obtaining information on the presence or absence of a cancer cell derived from colon cancer in a biological sample collected from a subject. The present invention also relates to use of a kit in the method.

## BACKGROUND ART

Colon cancer is a collective term for carcinoma located in large intestine (colon, rectum, anus). Colon cancer develops at the surface of intestinal mucosa, invades into the large intestinal wall and finally metastasizes to lymph node or other organs. Screening for colon cancer based on occult blood tests has been widely carried out because early detection of colon cancer may lead to remission of the disease. However, the examination of colon cancer is based on bleeding from the lesion site, and thus there is a possibility that colon cancer which is not accompanied by bleeding may be overlooked. The examination may also give positive results for bleeding due to diseases other than cancer such as hemorrhoids. Thus thorough examination of colon cancer is generally carried out by endoscopy that allows direct observation of the whole intestine. Meanwhile endoscopic examinations put a great burden on subjects because the examinations require dietary restriction as well as pre-treatment using laxatives and an endoscope is inserted into the intestine.

Examination for colon cancer includes measurement of tumor markers in blood. The tumor markers used for evaluation of metastasis and recurrence and evaluation of treatment efficacy of cancer include CEA (carcinoembryonic antigen), CA19-9 and the like. However, the examination of the tumor markers which has been utilized for examinations of types of cancer different from colon cancer has insufficient sensitivity and specificity as the specific screening examination for colon cancer.

Meanwhile, new diagnosis methods for cancer have been recently studied which are based on genetic information. The methods include, for example, ones based on information on methylation of DNAs. The methods use markers which are CpG sites (5'-(CG)-3') in base sequences of certain genes. Based on the analysis results of the methylation status of the markers, information such as presence or absence of a cancer cell is obtained, which information may be used as an index for diagnosis of cancer.

Methods for determining cancer utilizing methylation analyses of DNA have been studied and developed for colon cancer. For example, the publication by Hibi K. et al. discloses that genes such as p16 (also referred to as CDKN2A) are highly methylated in tissues from patients with colon cancer (see Non-Patent Literature 1). The publication by Qi J. et al. discloses that SFRP genes (SFRP1, SFRP2, SFRP4 and SFRP5) are hardly methylated in normal colon tissues while they are highly methylated in colon cancer tissues (see Non-Patent Literature 2). The publication by Vilkin A. et al. discloses that MLH1 gene is highly methylated in colon cancer tissues with microsatellite instability (see Non Patent Literature 3). Kim et al. (see Non Patent Literature 4) analyzed the methylation profile of 27578 CpG sites spanning more than 14000 genes in colorectal cancer and in the adjacent normal mucosa with bead-chip array-based technology, and identified 621 CpG sites located in promoter regions and CpG islands that were greatly hypermethylated in colorectal cancer compared to normal mucosa. US patent application 2012/196756 relates to the identification of regions of specific genes and specific regions of genomic nucleic acid useful in the detection of methylation associated with colorectal cancer. An exhaustive list of possible colorectal neoplasm-specific markers is disclosed. No comparison is made of methylation in colorectal cancer with other types of cancer. Kim et al. (see Non Patent Literature 5) discloses an overview of DNA methylation markers in colorectal cancer. Citation List

Non-Patent Literature

Non-Patent Literature 1: Hibi K. et al., Jpn. J. Cancer Res. vol. 93, p.883-887 (2002)
Non-Patent Literature 2: Qi J. et al., World J. Gastroenterol. vol. 12, p.7113-7117 (2006)
Non-Patent Literature 3: Vilkin A. et al., Cancer. vol. 115, p.760-769 (2009)
Non-Patent Literature 4 : Kim Y-H. et al., Annals of surgical oncology 18(8):2338-2347 (2011)
Non-Patent Literature 5 : Kim M.S. et al. Cancer and metastasis reviews29(1): 181-206 (2010)


## SUMMARY OF THE INVENTION

Although some genes indicative of abnormal methylation in colon cancer have been reported as described above, these genes contain those which are methylated in some extent in a type of cancer different from colon cancer. When methylation analysis is carried out with a marker that is methylated in several types of cancer including colon cancer, information deduced from the analysis result may include not only information on colon cancer but also information on other types of cancer. In this case, even when an analysis result provides information indicating that a sample contains a cancer cell for example, it is difficult to determine whether the cancer cell is derived from colon cancer or from other types of cancer.

As such upon obtaining information on colon cancer by methylation analysis of a marker, the specificity of the marker to colon cancer is very important. Thus there is a need for a novel marker specific to colon cancer, which allows specific detection of a cancer cell derived from colon cancer.

Accordingly an object of the present invention is to provide a method for obtaining information on the presence or absence of a cancer cell derived from colon cancer in a biological sample collected from a subject by identifying such a novel marker and analyzing methylation status of the marker. Another object of the present invention is the use of a kit for the method.

The inventors of the present invention have identified novel markers which are genetic regions specifically methylated in DNAs obtained from cancerous tissues of colon cancer. The inventors of the present invention have found that cancer cells derived from colon cancer can be clearly discriminated from other cells (cells of normal tissues, cells of non-cancerous tissues and cancer cells derived from a type of cancer other than colon cancer) based on the result obtained by analyzing methylation status of the markers, thereby completing the present invention.

Thus the present invention provides a method for obtaining information on the presence or absence of a cancer cell derived from colon cancer in a biological sample collected from a subject comprising the steps of:

preparing a DNA sample from the biological sample collected from the subject;
analyzing methylation status of a CpG site in a promoter region of at least one gene selected from ZNF304 (Zinc finger protein 304) and ZNF264 (Zinc finger protein 264) in the DNA sample obtained from the preparation step; and
obtaining information on the presence or absence of a cancer cell derived from colon cancer in the biological sample collected from the subject based on an analysis result obtained from the analyzing step.

The present invention also provides the use of a kit in said method for obtaining information on the presence or absence of a cancer cell derived from colon cancer in a biological sample collected from a subject, said kit comprising a primer set for analysis of methylation status of at least one CpG site selected from CpG sites located in promoter regions of ZNF304 and ZNF264 genes.

## BRIEF DESCRIPTION OF THE DRAWINGS

Fig. 1A is a graph showing the methylation positive rate of the promoter region of ZNF304 gene calculated from methylation data of cancerous tissues of colon cancer and non-cancerous colonic mucosa tissues;
Fig. 1B is a graph showing the methylation positive rate of the promoter region of ZNF264 gene calculated from methylation data of cancerous tissues of colon cancer and non-cancerous colonic mucosa tissues;
Fig. 2A is a graph showing the methylation positive rate of the promoter region of ZNF304 gene calculated from methylation data of various clinical specimens;
Fig. 2B is a graph showing the methylation positive rate of the promoter region of ZNF264 gene calculated from methylation data of various clinical specimens;
Fig. 3A is a graph showing the methylation positive rate of the promoter region of a known marker gene SFRP1 calculated from methylation data of various clinical specimens;
Fig. 3B is a graph showing the methylation positive rate of the promoter region of a known marker gene MLH1 calculated from methylation data of various clinical specimens;
Fig. 3C is a graph showing the methylation positive rate of the promoter region of a known marker gene CDKN2A calculated from methylation data of various clinical specimens;
Fig. 4A is a graph showing the methylation score of the promoter region of ZNF304 gene in DNAs extracted from various clinical specimens;
Fig. 4B is a graph showing the methylation score of the promoter region of ZNF264 gene in DNAs extracted from various clinical specimens;
Fig. 5 is an image showing the results of amplification by methylation specific PCR (MSP) of DNAs extracted from normal colonic mucosa tissues, non-cancerous colonic mucosa tissues and cancerous tissues of colon cancer using the respective primer sets for ZNF304 and ZNF264;
Fig. 6 is a graph showing the methylation positive rate of the promoter region of ZNF304 gene calculated from methylation data of cancerous tissues of colon cancer and non-cancerous tissues;

Fig. 7 is a graph showing the methylation positive rate of the promoter region of ZNF304 gene calculated from methylation data of various clinical specimens;

Fig. 8A is a graph showing the methylation positive rate of the promoter region of a known marker gene CDKN2A calculated from methylation data of various clinical specimens;

Fig. 8B is a graph showing the methylation positive rate of the promoter region of a known marker gene MLH1 calculated from methylation data of various clinical specimens;

Fig. 9 is an image showing the results of amplification by MSP of DNAs extracted from normal colonic mucosa tissues and cancerous tissues of colon cancer and non-cancerous tissues using a primer set for ZNF304 (SEQ ID NOs: 19 and 20);

Fig. 10A is a boxplot generated based on the corrected methylation score of the promoter region of ZNF304 gene in DNAs extracted from cancerous tissues of colon cancer and non-cancerous tissues;

Fig. 10B is a boxplot generated based on the corrected methylation score of the promoter region of ZNF264 gene in DNAs extracted from cancerous tissues of colon cancer and non-cancerous tissues;

Fig. 11A is an ROC curve obtained by ROC analysis using the corrected methylation score of the promoter region of ZNF304 gene;

Fig. 11B is an ROC curve obtained by ROC analysis using the corrected methylation score of the promoter region of ZNF264 gene;

Fig. 12A is a bar graph showing the DNA copy number after amplifying DNAs extracted from peripheral blood of colon cancer patients and healthy subjects by quantitative MSP with a primer set for ZNF304 (SEQ ID NOs: 28 and 29) and a fluorescent probe (SEQ ID NO: 30);

Fig. 12B is a bar graph showing the DNA copy number after amplifying DNAs extracted from peripheral blood of colon cancer patients and healthy subjects by MSP with a primer set for ZNF264 (SEQ ID NOs: 24 and 25) and a fluorescent probe (SEQ ID NO: 26);

Fig. 12C is a bar graph showing the band intensity of amplification products obtained by MSP of DNA extracted from peripheral blood of colon cancer patients and healthy subjects with a primer set for accuracy control (SEQ ID NOs: 21 and 22);

Fig. 13 is a bar graph showing the band intensity of amplification products obtained by MSP of DNA extracted from peripheral blood of colon cancer patients and healthy subjects with a primer set for ZNF304 (SEQ ID NOs: 32 and 33);

Fig. 14 is a schematic view of an example of a determination device for providing information on colon cancer in a subject;

Fig. 15 is a block diagram showing the functionality configuration of the determination device of Fig. 14;

Fig. 16 is a block diagram showing the hardware configuration of the determination device shown in Fig. 14; and

Fig. 17 is a flow chart of determination for providing information on colon cancer in a subject using the determination device in Fig. 14.

## MODES FOR CARRYING OUT THE INVENTION

In the method for obtaining information on colon cancer described herein (hereinafter also merely referred to as "method"), a DNA sample is first prepared from a biological sample collected from a subject.

The biological sample is not particularly limited as far as it is a biological sample containing DNA of a subject and is preferably a sample containing a genomic DNA such as a clinical specimen. The clinical specimen may include, for example, body fluid, urine, tissues obtained by operations or biopsies and the like. The body fluid may include blood, serum, plasma, lymph fluid, ascetic fluid, bone marrow aspirate, nipple discharge and the like. The biological sample may also be a culture medium obtained by culturing cells or tissues collected from a subject.

The DNA sample can be prepared by extracting DNA from the biological sample. The method for extracting DNA from biological samples is well known in the art. Extraction of DNA can be carried out, for example, by mixing the biological sample with a treatment solution containing a surfactant for solubilization of cells or tissues (e.g. sodium cholate, sodium dodecyl sulfate etc.), and subjecting the resulting mixture to physical procedure (stirring, homogenization, ultrasonication etc.) to release DNA contained in the biological sample into the mixture. In this case, it is preferable to centrifuge the mixture to precipitate cell debris and use the supernatant containing the released DNA to the next step of analyzing. The obtained supernatant may be purified according to well-known methods. DNA can also be extracted and purified from a biological sample by using commercially available kits.

The preparation step preferably further comprises the step of fragmenting the extracted DNA. By fragmenting DNA to have appropriate length, methylated DNA immunoprecipitation (MeDIP) and non-methylated cytosine conversion as described hereinbelow can be effectively carried out.

Fragmentation of DNA may be carried out by ultrasonication, alkaline treatment, restriction enzyme treatment and the like. When DNA is fragmented by alkaline treatment, a sodium hydroxide solution may be added to a DNA solution to the final concentration of 0.1N to 1.0N and the mixture may be incubated at 10°C to 40°C for 5 to 15 minutes to fragment the DNA. When the restriction enzyme treatment is used for DNA fragmentation, the restriction enzyme may appropriately be selected based on the base sequence of DNA, which may be *Mse*I or *Bam*HI, for example.

According to the present method, methylation status of a CpG site in a promoter region of at least one gene selected from ZNF304 and ZNF264 in DNA obtained from the preparation step is analyzed.

As used herein, the term "CpG site" means a site of a sequence in which cytosine (C) and guanine (G) are adjacent in this order from 5' to 3'. The letter "p" in "CpG" represents a phosphodiester bond between cytosine and guanine.

As used herein, to "analyze methylation status" means to analyze presence or absence of methylation of a CpG site located in a promoter region of at least one gene selected from ZNF304 and ZNF264 or analyze methylation frequency in the promoter region.

The base sequences *per se* of the promoter regions of ZNF304 and ZNF264 genes are well known in the art. The base sequences can be obtained from well-known databases such as the one provided by the National Center for Biotechnology Information (NCBI) (http://www.ncbi.nlm.nih.gov/). The ID numbers of the above genes are shown in Table 1. The base sequences of the promoter regions of the genes (sequences of negative strands) are represented by SEQ ID NOs: 1 and 2, respectively.

Table 1

| **Gene symbol** | Unigene ID | Entrez Gene ID | Transcript ID | **SEQ ID NO:** |
|---|---|---|---|---|
| *ZNF304* | Hs.287374 | 57343 | NM_020657.2 | 1 |
| *ZNF264* | Hs.515634 | 9422 | NM_003417.4 | 2 |

In embodiments of the present method, the analyzing step may be the step of analyzing presence or absence of methylation of at least one CpG site among CpG sites located in a promoter region of at least one gene selected from ZNF304 and ZNF264. The term "presence or absence of methylation" means whether or not cytosine in a CpG site located in the promoter region is methylated. One CpG site may be analyzed; however, more than one CpG site is preferably analyzed for presence or absence of methylation. More than one CpG site may be selected from a promoter region of one gene or from each of promoter regions of more than one gene.

In a further embodiment of the present method, the analyzing step may be the step of analyzing methylation frequency in a promoter region of at least one gene selected from ZNF304 and ZNF264. The term "methylation frequency" means the ratio of the number of methylated CpG site(s) relative to the number of CpG site(s) located in the promoter region. The target for analysis may be the whole promoter region or a part thereof including at least one CpG site. The target for analysis may contain only one CpG site; however it is preferable that the target for analysis contains more than one CpG site. The target for analysis may be selected from any one promoter region among the above genes or from promoter regions of more than one gene.

The positions and numbers of CpG sites located in the promoter regions of ZNF304 and ZNF264 genes are already known. Thus the number of methylated CpG site(s) itself in the promoter regions can also be used as the methylation frequency.

The methylation frequency may be "methylation score" obtained by analyzing DNA for methylation status of CpG sites with mass spectrometry such as MassARRAY® as described hereinbelow. MassARRAY® allows calculation of methylation score based on the ratio between the area of a peak derived from a methylated DNA fragment and the area of a peak derived from a non-methylated DNA fragment obtained after measurement of the DNA fragments.

The target for analysis may be any CpG site(s) (or certain region(s) including the CpG site(s)) in the promoter regions of ZNF304 and ZNF264 genes without particular limitation and may be appropriately selected by a person skilled in the art. The positions and numbers of CpG sites located in the promoter regions of the genes are already known. Thus the target CpG site or region may be selected by routine experiments according to the well known analysis methods described hereinbelow.

Various methods are well-known in the art as to the method for analyzing methylation status. According to the present method, it is not specifically limited as to which analysis method is used; however, the analysis method preferably comprises differentiating methylated DNA from non-methylated DNA, amplifying DNA and detecting methylated DNA and/or non-methylated DNA.

The step of differentiating methylated DNA from non-methylated DNA may include the step of carrying out methylation sensitive restriction enzyme treatment, the MeDIP method, non-methylated cytosine converting treatment and the like.

The step of amplifying DNA may include the step of carrying out PCR, quantitative PCR, IVT (*in vitro* transcription) amplification, SPIA™ amplification and the like methods.

The step of detecting methylated DNA and/or non-methylated DNA may include the step of carrying out electrophoresis, sequence analysis, microarray analysis, mass spectrometry, Southern hybridization and the like.

The MeDIP method is a method in which methylated DNA in a biological sample is concentrated by immunoprecipitation using an anti-methylated cytosine antibody or an anti-methylated cytidine antibody, or an antibody which specifically recognizes a methylated DNA-binding protein. In embodiments of the present method, the analyzing step may be the step of concentrating methylated DNA in DNA obtained in the extracting step by the MeDIP method and analyzing methylation status of the concentrated methylated DNA. The methylated DNA concentrated by the MeDIP method may be amplified by e.g. IVT amplification and methylation status of the obtained amplified product may be analyzed by using a microarray. These analysis procedures are referred to as the MeDIP on chip method.

The non-methylated cytosine converting treatment is the one in which DNA extracted from a biological sample is subjected to reaction with a non-methylated cytosine conversion agent so as to convert non-methylated cytosine(s) in the DNA to a different base (uracil, thymine, adenine or guanine). In this context, the non-methylated cytosine conversion agent is an agent which can react with DNA and convert a non-methylated cytosine in the DNA to a different base (uracil, thymine, adenine or guanine). The non-methylated cytosine conversion agent suitably used may be, for example, bisulfite such as sodium, potassium, calcium or magnesium bisulfite.

In the treatment using bisulfite, non-methylated cytosine(s) in DNA is converted to uracil due to deamination reaction, while a methylated cytosine does not undergo such a base conversion.

Thus, the difference in methylation status of a CpG site in DNA is converted to the difference in base sequence (C and U) by the non-methylated cytosine converting treatment using bisulfite. The non-methylated cytosine converting treatment using bisulfite is referred to as bisulfite treatment.

When the bisulfite treatment is carried out, the amount (concentration) of bisulfite added is not specifically limited so long as it can sufficiently convert non-methylated cytosine(s) in DNA, and corresponds to, for example, 1M or higher, preferably 1M to 15M, more preferably 3M to 10M as the final concentration in a solution containing DNA. The incubation conditions (temperature and time) after addition of bisulfite may be appropriately selected according to the amount added of bisulfite, and for example, when bisulfite is added at a final concentration of 6M, the incubation is carried out at 50°C to 80°C for 10 minutes to 90 minutes.

Methylation status of CpG sites in DNA can be analyzed by analyzing the sequence of DNA after bisulfite treatment and detecting the difference in base sequence from the original sequence. This process is referred to as bisulfite sequencing method.

Methylation status of CpG sites can be alternatively analyzed by mass spectrometry. Specifically, a DNA after bisulfite treatment as a template is amplified by PCR using a primer set specific for a base sequence which is a target for analysis, and the obtained PCR product is subjected to IVT amplification to convert methylated cytosine and uracil respectively to guanine (G) and adenine (A). The obtained IVT amplification product is digested with RNase A and the difference in mass (16 Da) due to difference between G and A in the obtained digested fragments is detected on a MALDI-TOF (matrix assisted laser desorption/ionization time-of-flight) mass spectrometer to analyze methylation status of DNA. This method is referred to as MassARRAY® analysis.

It is known that the cleavage site in IVT products by RNase A is between an arbitrary base and the adjacent uracil (U) or thymine (T). Thus the base sequence and mass of the IVT product cleaved by RNase A may be predicted based on the base sequence of the template DNA. Accordingly the peaks obtained in MassARRAY® can be identified as to the portions of the base sequences in the template DNA from which the peaks are originated. For example, when one CpG site is methylated in a DNA fragment, a peak obtained in MassARRAY® shifts to the side with an increased mass for 16 Da. When a DNA fragment containing more than one CpG site is analyzed, for example, the DNA fragment having two methylated CpG sites shows a shift of 32 Da and the DNA fragment having three methylated CpG sites shows a shift of 48 Da.

In mass spectrometry such as MassARRAY®, the methylation score of the analyzed DNA fragment can be calculated. For example when a DNA fragment having a certain sequence results in the ratio between the area of the peaks of non-methylated DNA fragments and the area of the peaks of methylated DNA fragments obtained in a resulting chart from the analysis of 1:3, the methylation score of the DNA fragment is 0.75 (= 3/(1+3)). The methylation score is theoretically 1 for a DNA fragment in which all CpG site(s) is methylated and 0 for a DNA fragment without any methylated CpG site.

Methylation status of CpG sites can be analyzed by a methylation specific PCR (MSP) method. The MSP method is a method in which methylation status of CpG sites (presence or absence of methylation) is analyzed by amplifying DNA after bisulfite treatment by PCR using a primer set described hereinafter and determining presence or absence of a PCR product.

The MSP method utilizes a primer set which can amplify a base sequence having a CpG site to be analyzed that is methylated (i.e. cytosine is not converted to uracil) but cannot amplify a base sequence having a CpG site that is not methylated (i.e. cytosine is converted to uracil). According to the MSP method using such a primer set, presence of a PCR product indicates methylation of the CpG site analyzed.

The MSP method may also be carried out by using a primer set which cannot amplify a base sequence having cytosine in a CpG site to be analyzed that is not converted to uracil but can amplify a base sequence having cytosine in a CpG site that is converted to uracil. In this case, absence of a PCR product indicates methylation of the CpG site analyzed.

Each primer in the primer set used for the MSP method may be appropriately designed by a person skilled in the art based on the base sequence including a CpG site to be analyzed, and it is preferably designed so as to contain cytosine of the CpG site to be analyzed at the 3' end or in the vicinity thereof of the primer.

Methylation status of CpG sites may alternatively be analyzed with a microarray. In this case, the microarray for analysis may be prepared by immobilizing a nucleic probe complementary to the base sequence of a promoter region of ZNF304 or ZNF264 gene on a substrate. The microarray can be prepared according to well-known methods in the art.

In the analysis using a microarray, DNA extracted from a biological sample is preferably labeled with a labeling substance well-known in the art. Thus, the present method preferably further comprises the step of labeling the extracted DNA. The labeling step is advantageously carried out after the DNA amplifying step because all DNA in the biological sample may be labeled. The labeling substance may include fluorescence substances, haptens such as biotin, radioactive substances and the like. The fluorescence substances may include Cy3, Cy5, FITC, Alexa Fluor™ and the like. Labeling of DNA facilitates measurement of a signal from a probe on the microarray. A method for labeling DNA with the labeling substance is well-known in the art.

The above signal may be any suitable signal depending on the type of microarrays. For example, the signal may be an electric signal generated when a DNA fragment hybridizes to a probe on the microarray, or a fluorescence or luminescence signal generated from a labeling substance when DNA to be analyzed is labeled as described above. Detection of a signal can be carried out by using a scanner comprised in a conventional microarray analyzer. The scanner may be, for example, GeneChip® Scanner3000 7G (Affymetrix), Illumina® BeadArray Reader (Illumina) and the like.

In the present method, information on colon cancer in the subject is obtained based on the analysis result obtained in the analyzing step. The information on colon cancer is not particularly limited as far as it may be an index on diagnosis of colon cancer and is preferably information indicative of occurrence or status of colon cancer or both thereof in a subject. The information may include preferably presence or absence of a cancer cell derived from colon cancer in a biological sample collected from a subject. The information may also include e.g., a possibility of occurrence of colon cancer in a subject, or a risk for future occurrence of colon cancer in a subject. The information on colon cancer in a subject who has already been affected by colon cancer may include prognosis of the subject, a degree of progression (stage) and the like. The information on colon cancer obtained based on the analysis result obtained in the analyzing step does not substantially include information on a type of cancer different from colon cancer because the promoter regions of the above genes which are used as markers in the present method are specifically methylated in cancer cells derived from colon cancer.

In embodiments of the present method, the analyzing step which provides the analysis result indicating presence of a methylated CpG site may provide information indicating occurrence of colon cancer or indicating that the status of colon cancer is poor (or aggravated).

In a further embodiment of the present method, the above information can be obtained when the methylation frequency obtained in the analyzing step is at or higher than a certain threshold.

More specifically, the information obtained may be indicative of presence of a cancer cell derived from colon cancer in a biological sample. The information obtained may alternatively indicate that a subject has a high risk for being affected by colon cancer or that a subject has already been affected by colon cancer. For a subject who has already been affected by colon cancer, information obtained may indicate that prognosis of the subject is poor (or aggravated) or that the cancer is in a progressed stage.

On the contrary, when the result from the analyzing step shows absence of methylated CpG site, information suggesting no occurrence of colon cancer or information indicating that colon cancer is in a preferable status can be obtained. Alternatively the above information can be obtained when the methylation frequency obtained in the analyzing step is lower than a certain threshold. More specifically, the information obtained may be indicative of absence of a cancer cell derived from colon cancer in a biological sample. The information obtained may alternatively indicate that a subject has a low risk for being affected by colon cancer or that a subject has not been affected by colon cancer. For a subject who has already been affected by colon cancer, information obtained may be indicative of a preferable prognosis of the subject or indicate that the cancer is in a relatively early stage.

The threshold is not particularly limited and may be empirically established based on accumulated data on various biological samples. The threshold may alternatively be established as follows. First, methylation frequency is analyzed for DNA extracted respectively from a biological sample which is confirmed to be devoid of cancer cells derived from colon cancer (normal colon tissue or normal colonocyte) and a biological sample containing a cancer cell derived from colon cancer. Next, based on the obtained analysis results, a threshold is established within a range that is higher than the methylation frequency of the biological sample devoid of cancer cells and lower than that of the biological sample containing the cancer cell. Preferably, the threshold is established as a value which can highly accurately differentiate between the biological sample devoid of cancer cells and the biological sample containing the cancer cell.

Also disclosed herein is a marker for obtaining information on colon cancer by methylation analysis (also merely referred to as "marker"). The marker is at least one CpG site selected from CpG sites located in promoter regions of ZNF304 and ZNF264 genes.

Methylation status of the marker in a DNA sample prepared from a biological sample collected from a subject may be analyzed and information on colon cancer in the subject can be obtained based on the analysis result. The analysis of methylation status and obtainment of information on colon cancer are the same as those previously described herein.

The scope of the present invention encompasses the use of a kit in the method for obtaining information on colon cancer provided herein. The kit comprises a primer set for analysis of methylation status of at least one CpG site selected from CpG sites located in promoter regions of ZNF304 and ZNF264 genes.

The primer set in the kit may be a primer set for analysis of methylation status of CpG sites according to mass spectrometry such as MassARRAY® or an analysis method involving PCR amplification such as the MSP method, the bisulfite sequencing method, among which the primer set for mass spectrometry such as MassARRAY® or for the MSP is preferred. The base sequences of the primers in the primer set may be appropriately selected by a person skilled in the art based on the base sequences in the promoter regions. Examples of the primer set include a primer set of primers having base sequences SEQ ID NOs: 3 and 4; a primer set of primers having base sequences SEQ ID NOs: 5 and 6; a primer set of primers having base sequences SEQ ID NOs: 9 and 10; a primer set of primers having base sequences SEQ ID NOs: 11 and 12; a primer set of primers having base sequences SEQ ID NOs: 19 and 20; and a primer set of primers having base sequences SEQ ID NOs: 32 and 33.

Examples of the combination of a primer set and a fluorescent probe for MSP by quantitative PCR include a combination of a primer set of primers having base sequences SEQ ID NOs: 24 and 25 and a doubly labeled probe having a base sequence SEQ ID NO: 27; and a combination of a primer set of primers having base sequences SEQ ID NOs: 28 and 29 and a doubly labeled probe having a base sequence SEQ ID NO: 30. The doubly labeled probe may be, for example, a probe labeled with a fluorescence substance at one end and with a quencher substance at the other end. Doubly labeled probes generally available include TaqMan® probe and the like.

Also disclosed herein is a system suitable for provision of information on colon cancer in a subject. For example, the system may be as follows.

A system suitable for provision of information on colon cancer in a subject comprising a computer containing a processor and a memory controlled by the processor, wherein the memory comprises a computer program for enabling the computer to carry out the steps of:

obtaining an analysis result on methylation status of a CpG site located in a promoter region of at least one gene selected from ZNF304 and ZNF264 in a DNA sample derived from the subject; and
providing information on colon cancer in the subject based on the resulting analysis result.

Also disclosed herein is a computer program product for enabling a computer to carry out provision of information on colon cancer in a subject. For example, the computer program product may be as follows.

A computer program product for enabling a computer to carry out provision of information on colon cancer in a subject comprising a computer readable medium, wherein the medium comprises a computer program for enabling the computer to carry out the steps of:

obtaining an analysis result on methylation status of a CpG site located in a promoter region of at least one gene selected from ZNF304 and ZNF264 in a DNA sample derived from the subject; and
providing information on colon cancer in the subject based on the resulting analysis result.

An example of a suitable device for carrying out the present method is illustrated hereinafter by referring to figures. Fig. 14 is a schematic view of an example of a determination device for providing information on colon cancer in a subject. The determination device 1 shown in Fig. 14 comprises a measurement device 2 and a computer system 3 connected to the measurement device 2.

In the present example, the measurement device 2 is a MALDI-TOF mass spectrometer. The measurement device 2 obtains mass spectrometric information such as the time of flight or the mass-to-charge ratio (m/z ratio) of a substance to be analyzed. The measurement device 2 onto which a measurement sample prepared from the DNA sample derived from a subject is mounted obtains mass spectrometric information of a nucleic acid in the measurement sample and sends the mass spectrometric information to the computer system 3.

The measurement device 2 may be, when methylation status is analyzed by the MSP method, a gel imaging device such as a fluorescence image scanner. In this case, the measurement device 2 onto which a gel obtained by electrophoresis of a reaction solution after nucleic acid amplification by the MSP method is mounted detects amplification products. The measurement device 2 then obtains the band intensity data of the amplification products and sends the obtained data to the computer system 3.

The computer system 3 comprises a computer main body 3a, an input device 3b and a display 3c which displays specimen information, determination results and the like. The computer system 3 receives the mass spectrometric information from the measurement device 2. The processor in the computer system 3 executes, based on the mass spectrometric information, a program for providing information on colon cancer in a subject.

Fig. 15 is a block diagram showing the functionality configuration of the determination device of Fig. 14. As shown in Fig. 15, the computer system 3 comprises a receiving unit 301, a memory unit 302, a calculating unit 303, a determining unit 304 and an output unit 305. The receiving unit 301 is communicably connected to the measurement device 2 though a network. The calculating unit 303 and the determining unit 304 are included in a control unit 306.

The receiving unit 301 obtains information from the measurement device 2. The memory unit 302 stores a threshold necessary for determination and a formula for calculating the methylation score. The calculating unit 303 calculates the methylation score from information obtained at the receiving unit 301 according to the formula stored in the memory unit 302. The determining unit 304 determines whether or not the methylation score calculated at the calculating unit 303 is lower than the threshold stored at the memory unit 302. The output unit 305 outputs the determination result from the determining unit 304 as information on colon cancer in the subject (e.g., presence or absence of a cancer cell derived from colon cancer in the biological sample collected from the subject).

Fig. 16 is a block diagram showing the hardware configuration of the determination device in Fig. 14. As shown in Fig. 16, the computer main body 3a comprises a CPU (Central Processing Unit) 30, ROM (Read Only Memory) 121, ROM 32, a hard disk 33, an input/output interface 34, a read-out device 35, a communication interface 36 and an image output interface 37. The CPU 30, ROM 31, RAM (Random Access Memory) 32, the hard disk 33, the input/output interface 34, the read-out device 35, the communication interface 36 and the image output interface 37 are connected via a bus 38 so as to be able to communicate with each other.

The CPU 30 can execute a computer program stored in ROM 31 and a computer program loaded with ROM 32. When the CPU 30 executes the application program, the functional blocks described above may be executed. Accordingly the computer system serves as a terminal that is a determination device for providing information on colon cancer in a subject.

ROM 31 is made up with mask ROM, PROM, EPROM, EEPROM or the like. ROM 31 stores the computer program executed by the CPU 30 and data used for the execution.

ROM 32 is made up with SRAM, DRAM or the like. ROM 32 is used for readout of the computer programs stored in ROM 31 and the hard disk 33. ROM 32 is also utilized as a work area when the CPU 30 executes these computer programs.

An operating system to be executed by the CPU 30, computer programs such as application programs (the computer program for providing information on colon cancer in a subject) and data for executing the computer programs are installed on the hard disk 33.

The read-out device 35 is made up with a flexible disk drive, a CD-ROM drive, a DVD-ROM drive or the like and can read out the computer program or data stored on a portable memory medium 40.

The input/output interface 34 is made up with a serial interface such as USB, IEEE1394 and RS-232C, a parallel interface such as SCSI, IDE and IEEE1284 and an analog interface formed by a D/A converter, an A/D converter or the like. The input/output interface 34 is connected to the input device 3b such as a keyboard and a mouse. A user can input data into the computer main body 3a by means of the input device 3b.

The communication interface 36 is, for example, an Ethernet® interface. The computer system 3 can send printing data to a printer via the communication interface 36.

The image output interface 37 is connected to the display 3c made up with a LCD, a CRT and the like. Accordingly the display 3c can output an image signal according to image data provided by the CPU 30. The display 3c displays an image (on a screen) according to the input image signal.

The process operations carried out by the determination device 1 for providing information on colon cancer in a subject are illustrated hereinafter. Fig. 17 is a flow chart for providing information on colon cancer using the determination device of Fig. 14. An example is herein illustrated in which mass spectrometric information of a nucleic acid in a measurement sample prepared from a DNA sample derived from a subject is used for calculation of a peak area from which a methylation score is calculated and the methylation score is determined as to whether or not it is lower than a threshold. In the step S1-1, the receiving unit 301 in the determination device 1 receives from the measurement device 2 mass spectrometric information. In the next step S1-2, the calculating unit 303 calculates from the mass spectrometric information received at the receiving unit 301 a peak area which is sent to the memory unit 302. In the step SI-3, the calculating unit 303 calculates the methylation score based on the peak area stored in the memory unit 302 according to the formula stored in the memory unit 302.

In the step SI-4, the determining unit 304 determines whether or not the methylation score calculated at the calculating unit 303 is lower than the threshold stored in the memory unit 302. When the methylation score is lower than the threshold, the determining unit 304 in the step S1-5 sends a determination result indicating that the biological sample collected from the subject does not contain a cancer cell derived from colon cancer to the output unit 305. When the methylation score is not lower than the threshold (i.e., the methylation score is at or higher than the threshold), the determining unit 304 sends a determination result indicating that the biological sample collected from the subject contains a cancer cell derived from colon cancer to the output unit 305.

In the step S1-7, the output unit 305 outputs the determination result as information on colon cancer in the subject, so that the display 3c displays the result and/or the printer prints out the result. Accordingly, the determination device can provide, to a physician or the like, information assisting the physician or the like to judge whether or not the subject has colon cancer.

The present invention is specifically described hereinafter by way of Examples which do not limit the present invention.

**Examples**

Reference Example 1: Identification of novel markers utilizing methylation data of cancerous tissues and non-cancerous tissues from colon cancer

(1) Collection of methylation data

In the present Reference Example, methylation data on the Infinium HumanMethylation27 BeadChip (Illumina) for cancerous tissues of colon cancer (236 specimens) and non-cancerous colonic mucosa tissues (16 specimens) were collected which are available from TCGA (The Cancer Genome Atlas : http://tcga-data.nci.nih.gov/tcga/tcgaHome2.jsp).

(2) Identification of novel markers

The Infinium HumanMethylation27 BeadChip includes probes for methylated and non-methylated CpG sites for each of 27,578 CpG sites on the human genome. In the present Reference Example, the signal intensity (signal M) from probes for methylated CpG sites and the signal intensity (signal U) from probes for non-methylated CpG sites were detected on BeadArray Reader and the methylation rate (mCpG) of CpG sites in the respective genes was calculated according to the following calculation formula:

$$(\text{mCpG}) = (\text{signal M})/\{(\text{signal M}) + (\text{signal U})\}$$

The threshold was set as "0.4" for the methylation rate (mCpG) of genes, and a specimen having a methylation rate at or higher than the threshold was designated as "methylation positive specimen". Based on the number of methylation positive specimens, methylation positive rate (%) for the respective genes in various tissues was calculated according to the following formula:

$$\text{Methylation positive rate (\%)} = (\text{number of methylation positive specimens/total number of specimens}) \times 100$$

For example, for cancerous tissue specimens, the methylation positive rate of a gene can be calculated by "(number of methylation positive specimens among cancerous tissue specimens/total number of cancerous tissue specimens) × 100". The gene which showed a statistically significant difference between cancerous tissue specimens and non-cancerous tissue specimens was identified as a marker which is methylated in cancerous tissues.

As a result of data mining using the Infinium HumanMethylation27 BeadChip (Illumina), promoter regions of ZNF304 and ZNF264 genes were identified as markers which are highly methylated in cancerous tissues of colon cancer (see Figs. 1A and 1B). These markers may also be referred to as the present markers hereinbelow.

Reference Example 2: Comparison of methylation data between cancer/tumor tissue specimens derived from several types of cancer/tumor, non-cancerous tissue specimens and normal tissue specimens

(1) Collection of methylation data

In the present Reference Example, in addition to the cancerous tissues of colon cancer (236 specimens) and non-cancerous colonic mucosa tissues (16 specimens) described in Reference Example 1, methylation data of 9 types of cancer/tumor tissue specimens, 6 types of non-cancerous tissue specimens and 13 types of normal tissue specimens were compared. The number of specimens for the respective tissues is shown in the following tables.

Table 2

| Cancer/tumor tissue specimens | |
|---|---|
| **Tissue** | **No. of specimens** |
| **Brain tumor (Brain)** | 283 |
| **Breast cancer (Breast)** | 186 |
| **Lung cancer (Lung)** | 59 |
| **Gastric cancer (Gastric)** | 82 |
| **Hepatocellular carcinoma (Liver)** | 100 |
| **Renal cell carcinoma (Kidney)** | 219 |
| **Uterine cancer (Uterus)** | 70 |
| **Ovarian cancer (Ovary)** | 519 |
| **Prostate cancer (Prostate)** | 93 |

Table 3

| Non-cancerous tissue | |
|---|---|
| **Tissue** | **No. of specimens** |
| **Lung** | 59 |
| **Cirrhosis tissue (Liver)** | 39 |
| **Kidney** | 199 |
| **Uterus** | 1 |
| **Ovary** | 16 |
| **Prostate** | 87 |

Table 4

| Normal tissue | |
|---|---|
| **Tissue** | **No. of specimens** |
| **Normal brain (Brain)** | 2 |
| **Normal oral mucosa (Oral)** | 2 |
| **Normal lung (Lung)** | 2 |
| **Normal colonic mucosa (Colon)** | 2 |
| **Normal liver (Liver)** | 2 |
| Peripheral blood from healthy subject (Blood) | 2 |
| **Normal skeletal muscle (Skeletal)** | 2 |
| **Normal testis (Testis)** | 1 |
| **Normal gastric mucosa (Stomach)** | 2 |
| **Normal pancreas (Pancreas)** | 1 |
| **Normal spleen (Spleen)** | 1 |

(continued)

| Normal tissue | |
|---|---|
| **Tissue** | **No. of specimens** |
| **Normal kidney (Kidney)** | 2 |
| Peripheral blood from healthy subject (Blood)* (Blood, Sahia B, et al.) | 93 |

Among the above specimens, the methylation data for cancerous tissue specimens derived from gastric cancer, cancerous tissue specimens derived from hepatocellular carcinoma, non-cancerous tissue specimens derived from hepatocellular carcinoma, normal tissue specimens derived from normal liver and 12 types of normal tissue specimens were those measured by Infinium Methylation Assay using the Infinium HumanMethylation27 BeadChip (Illumina) in the same manner as described in Reference Example 1. The methylation data for other specimens were the methylation data of the Infinium HumanMethylation27 BeadChip (Illumina) available from TCGA (The Cancer Genome Atlas: http://tcga-data.nci.nih.gov/tcga/tcgaHome2.jsp) or, for the specimens of lung cancer and prostate cancer and 93 specimens of peripheral blood from healthy subjects, published in the following references. The methylation data in this context are the methylation rate (mCpG) of CpG sites in ZNF304 and ZNF264 obtained as described in section (2) in Reference Example 1.

- Lung cancer: Selamat SA et al., Genome-scale analysis of DNA methylation in lung adenocarcinoma and integration with mRNA expression. Genome Res. Jul; 22 (7): 1197-211 (2012).
- Prostate cancer: Kobayashi Y et al., DNA methylation profiling reveals novel biomarkers and important roles for DNA methyltransferases in prostate cancer. Genome Res. Jul 21 (7): 1017-27 (2011).
- Data of 93 specimens of peripheral blood from healthy subjects: Salhia B et al., DNA methylation analysis determines the high frequency of genic hypomethylation and low frequency of hypermethylation events in plasma cell tumors. Cancer Res. Sep 1; 70 (17): 6934-44 (2010).

(2) Comparison of methylation positive rate between cancer/tumor tissue specimens derived from several types of cancer/tumor, non-cancerous tissue specimens and normal tissue specimens

The threshold was set as "0.4" for the methylation rate (mCpG) of genes, and a specimen having a methylation rate at or higher than the threshold was designated as "methylation positive specimen". Based on the number of methylation positive specimens, methylation positive rate (%) for the present markers in various tissues was calculated according to the above formula. The results are shown in Figs. 2A and 2B. In Fig. 2, "Normal tissue" represents, among the tissues indicated in Table 4, the normal tissues excluding 93 specimens of peripheral blood from healthy subjects (Salhia B et al.) and "Normal blood" represents the 93 specimens of peripheral blood from healthy subjects (Salhia B et al.).

Figs. 2A and 2B show that both of the present markers are not highly methylated in other types of cancer or human normal tissues or human normal blood (peripheral blood from healthy subjects, Salhia B et al.) and thus are specifically and highly methylated in colon cancer.

Comparative Example 1: Comparison of methylation positive rate between cancer/tumor tissue specimens derived from several types of cancer/tumor, non-cancerous tissue specimens and normal tissue specimens

The methylation positive rate was calculated for each of SFRP1, MLH1 and CDKN2A genes (hereinafter referred to as "known markers") which have already been known to be methylated in cancer cells derived from colon cancer in the similar manner as Reference Example 2 in the respective tissues. The results are shown in Figs. 3A to 3C.

According to Figs. 3A to 3C, SFRP1, MLH1 and CDKN2A showed high positive rates in colon cancer and were also methylated in other types of cancer and other non-cancerous tissues. Therefore, these genes have low specificity towards colon cancer. Thus, the known markers have high sensitivity while having low specificity as markers of colon cancer, thereby posing issues in terms of performance as diagnostic markers of colon cancer. Namely it was shown that a search for markers is required to be carried out by taking the specificity in other types of cancer and other non-cancerous tissues into account.

Example 1: Comparison of methylation data (MassARRAY) between normal colonic mucosa, non-cancerous colonic mucosa and colon cancer

(1) Biological samples

In the present Example, the biological samples used were normal colonic mucosa tissues (2 specimens), non-cancerous colonic mucosa tissues (2 specimens) and cancerous tissues of colon cancer (5 specimens).

(2) Preparation of measurement samples and control samples

(i) Extraction of genomic DNA

Genomic DNA was extracted from the above tissues with the QIAamp DNA Mini Kit (QIAGEN). The genomic DNA contained in the obtained DNA solution was fragmented with Bioruptor (COSMO BIO). In order to generate a calibration curve for mass spectrometry, the control genomic DNA used was genomic DNA of human peripheral blood lymphocytes. The genomic DNA from human peripheral blood lymphocytes was amplified with the GenomiPhi v2DNA amplification kit (GE Healthcare Life Sciences). The obtained amplification product consisted of non-methylated DNA. The amplification product was fragmented with Bioruptor (COSMO BIO) to obtain a solution of non-methylated DNA fragments (0% methylated DNA). A portion of the solution of the non-methylated DNA fragments was subjected to reaction with SssI methylase (New England Biolab) to methylate all cytosines in CG sequences and obtain a solution of methylated DNA fragments (100% methylated DNA). By mixing the 0% methylated DNA solution and the 100% methylated DNA solution at certain proportions, solutions of 25%, 50% and 75% methylated DNA were obtained.

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 μl).

(iii) Amplification by PCR and IVT

Methylated cytosine and uracil in the DNA fragments after bisulfite treatment were converted respectively to guanine and adenine by PCR and IVT amplification.

It is verified by MassARRAY® analysis using control samples as described hereinbelow that the primer sets used for PCR can amplify both methylated DNA and non-methylated DNA without bias. The sequences of the primer sets for the present markers are shown in Table 5. The base sequences (sequences of negative strands) which can be analyzed with the primer sets shown in Table 5 in the promoter regions in ZNF304 and ZNF264 genes are shown in SEQ ID NOs: 13 and 14, respectively.

Table 5

| Marker | Base sequence of primer | SEQ ID NO: |
|---|---|---|
| *ZNF304* | **Forward** : TAATAATGAGTTGGGTATAGA | 3 |
| | **Reverse** : ACATACCCTTTAAAAACAAC | 4 |
| *ZNF264* | **Forward** : GTATAAATTAAGTGGTAAATT | 5 |
| | **Reverse** : CTCCTCTATCCCAACTCTAC | 6 |

The following tag sequence and T7 promoter sequence were respectively added to the 5'-terminals of the forward primers and reverse primers in the above primer sets for IVT reaction.

- Tag sequence: AGGAAGAGAG (SEQ ID NO: 7)
- T7 promoter sequence: CAGTAATACGACTCACTATAGGGAGAAGGCT (SEQ ID NO: 8)

PCR reaction solution was prepared by mixing the following reagents:

| | |
|---|---|
| 10 × Hot Star buffer (QIAGEN) | 0.5 μL |
| 25 mM dNTP mix | 0.04 μL |

(continued)

| | |
|---|---|
| Hot Star Taq (5 U/μL) (QIAGEN) | 0.04 μL |
| Primer mix | 2.0 μL |
| DNA solution | 1.0 μL |
| Water | 1.42 μL |
| Total | 5 μL |

PCR reaction was carried out on the reaction solution under the following conditions:

1 cycle of 94°C for 15 minutes;

45 cycles of 94°C for 20 seconds, 52°C for 30 seconds and 72°C for 1 minute; and

72°C for 3 minutes.

The obtained PCR products were dephosphorylated with SAP (Shrimp Alkaline Phosphatase) in the MassCLEAVE™ Reagent kit (SEQUENOM). The following reaction solution prepared with the kit was then added.

| | |
|---|---|
| 5 × T7 R&DNA polymerase buffer | 0.89 μL |
| T Cleavage mix | 0.24 μL |
| 100 mM DTT | 0.22 μL |
| T7 R&DNA polymerase | 0.44 μL |
| RNase A | 0.06 μL |
| RNase-free water | 3.15 μL |
| Total | 5 μL |

The obtained mixture was incubated at 37°C for 3 hours to effect IVT reaction and uracil or thymine specific cleavage. The obtained reaction product was purified with Clean Resin (SEQUENOM) to obtain a sample for mass spectrometry. Samples derived from genomic DNA extracted from the above clinical specimens were designated as measurement samples and samples derived from control genomic DNA were designated as control samples, both of which were used for mass spectrometry described hereinbelow.

(3) Analysis of methylation status by mass spectrometry using MassARRAY®

(i) Preparation of calibration curve

Two mass spectrometric analyses were carried out independently on the control samples obtained as above. On the basis of the obtained analysis results calibration curves were prepared for the respective primer sets and correlation coefficients were calculated. Accordingly it was verified that the primer sets used could amplify both methylated DNA and non-methylated DNA without bias.

(ii) Analysis of measurement samples

The measurement samples obtained as above were subjected to mass spectrometry to obtain peaks of DNA fragments contained in the measurement samples. The obtained peaks were then allocated to the portions of the base sequences of ZNF304 and ZNF264. The methylation score was calculated on the basis of the ratio between the area of the peaks of the fragments containing a methylated CpG site and the area of the peaks of the fragments without the methylated CpG site, both fragments having the same base sequence. The obtained results are shown in Fig. 4A and 4B.
According to Figs. 4A and 4B, it was found that ZNF304 and ZNF264 showed low methylation scores in non-cancerous colonic mucosa tissues and high methylation scores in colon cancer. It was therefore demonstrated that the methylation score of the present markers correlates with colon cancer, similar to the results of the Infinium method in Reference Example 1. In addition, according to Fig. 4, it was found that analysis of methylation frequency of the present markers allows establishment of the threshold that can distinguish specimens of colon cancer and specimens of normal colonic mucosa tissues and non-cancerous colonic mucosa tissues.

Example 2: Comparison of methylation data (MSP) between normal colonic mucosa, non-cancerous colonic mucosa and colon cancer

(1) Biological samples

In the present Example, the biological samples used were normal colonic mucosa tissues (2 specimens), non-cancerous colonic mucosa tissues (2 specimens) and cancerous tissues of colon cancer (5 specimens) as in Example 1.

(2) Preparation of measurement samples and control samples

(i) Extraction of genomic DNA

Genomic DNA was extracted from the above tissues with the QIAamp DNA Mini Kit (QIAGEN). The genomic DNA contained in the obtained DNA solution was fragmented with Bioruptor (COSMO BIO).

The control genomic DNA used was genomic DNA of human peripheral blood lymphocytes. The genomic DNA from human peripheral blood lymphocytes was amplified with the GenomiPhi v2DNA amplification kit (GE Healthcare Life Sciences). The obtained amplification product consisted of non-methylated DNA. The amplification product was fragmented with Bioruptor (COSMO BIO) to obtain a solution of non-methylated DNA fragments (0% methylated DNA). A portion of the solution of the non-methylated DNA fragments was subjected to reaction with SssI methylase (New England Biolab) to methylate all cytosines in CG sequences and obtain a solution of methylated DNA fragments (100% methylated DNA).

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 μl).

(3) Methylation specific PCR (MSP)

MSP was carried out on the measurement samples and control samples (DNA after bisulfite treatment) obtained in the above section (2). The composition of PCR reagents, primer sets and reaction conditions for PCR in MSP are shown below.

<PCR reagent>

| | |
|---|---|
| DDW (sterilized water) | 16.8 μL |
| 10 × PCR buffer with $MgCl_2$ (Roche) | 2.5 μL |
| 2 mM dNTP mix | 2.5 μL |
| 10 μM sense primer | 1.0 μL |
| 10 μM antisense primer | 1.0 μL |
| Faststart Taq polymerase (Roche) | 0.2 μL |
| Measurement sample or control sample | 1.0 μL |
| Total | 25 μL |

<Primer set>

The primer sets used for MSP are shown in Table 6. These primer sets allow generation of amplification products when DNA in the amplified regions is methylated. A primer set for accuracy control was also used which allows judgment on whether or not the bisulfite treatment had been appropriately carried out (see Table 7). The base sequences (sequences of negative strands) which can be analyzed with the primer sets shown in Table 6 in the promoter regions in ZNF304 and ZNF264 genes are shown in SEQ ID NOs: 17 and 18, respectively.

Table 6

| Gene symbol | Primer | Base sequence of primer | SEQ ID NO: | Size of PCR product (bp) | Annealing temp. (°C) | Cycles |
|---|---|---|---|---|---|---|
| *ZNF304* | ZNF304_MF | TTGTATTTAATTAGGTAACGAGCGT | 9 | 123 | 56 | 36 |
| | ZNF304_MR | TTTAAAAACAACTATAACCTCCGAA | 10 | | | |
| *ZNF264* | ZNF264_MF | ATTTATTTCGGAAATCGTTTTAGC | 11 | 124 | 60 | 36 |
| | ZNF264_MR | ACTGTACGACCCAAAAAATAACGTA | 12 | | | |

Table 7

| Base sequence of primer | | SEQ ID NO: | Size of PCR product (bp) | Annealing temp. (°C) | Cycles |
|---|---|---|---|---|---|
| **Forward** | GGGATATTAAGTGGAGTTATTTTGGTTTTAGTT | 15 | 129 | 60 | 40 |
| **Reverse** | CCCTCCCAACATCCTTCCTAA | 16 | | | |

<PCR reaction conditions>

95°C for 6 minutes;

Y cycles of 95°C for 30 seconds, X°C for 30 seconds and 72°C for 30 seconds;

72°C for 7 minutes; and

keep at 16°C.

In the above reaction conditions, "X" and "Y" respectively represent the annealing temperature and the number of cycles as indicated in Tables 6 and 7.

(4) Analysis of results of methylation specific PCR (MSP)

The amplification product from MSP was verified by 2% agarose gel electrophoresis. The results are shown in Fig. 5. In this figure, "0" and "100" under "control" represent the 0% methylation control sample and the 100% methylation control sample, respectively.

In PCR using the primer set for accuracy control, bands were detected in all lanes as shown in Fig. 5. This shows that bisulfite treatment of the samples was appropriately carried out. In PCR using the present markers, bands derived from methylated CpGs were not detected for any normal colonic mucosa tissues or non-cancerous colonic mucosa tissues. In contrast, PCR of colon cancer tissue samples resulted in detection of bands in 5 samples among 5 samples for both markers of ZNF304 and ZNF264. Accordingly it is indicated that in methylation analysis of the present markers by MSP method, methylation of the present markers and colon cancer are correlated, similar to the result of the Infinium method from Reference Example 1. Namely it is suggested that ZNF304 and ZNF264 are markers with high specificity such that they tend to be highly methylated in colon cancer but methylation thereof is not detected in other normal tissues.

Reference Example 3: Analysis of CpG sites in promoter region of ZNF304 gene

In the present Reference Example, CpG sites in the promoter region of ZNF304 gene were further analyzed by utilizing the methylation data on the Infinium HumanMethylation450 BeadChip (Illumina).

(1) Collection of methylation data

Methylation data on the Infinium HumanMethylation450 BeadChip (Illumina) for cancerous tissues of colon cancer (324 specimens) and non-cancerous tissues (40 specimens) were collected which are available from TCGA (The Cancer Genome Atlas : http://tcga-data.nci.nih.gov/tcga/tcgaHome2.jsp).

(2) Calculation of methylation positive rate of CpG site of ZNF304

The Infinium HumanMethylation450 BeadChip includes probes that allow quantification of methylation status of 482,421 CpG sites on human genome. The Infinium HumanMethylation450 BeadChip is designed to allow quantification of signal intensity of methylated DNA fragments and non-methylated DNA fragments for respective CpG sites by means of hybridization to probes and single base extension reaction. The 265,824 probes corresponding to about 55% of all probes target the regions within 5 kb from the transcription initiation sites of genes (10.1 probes per gene in average). In the present Reference Example, the signal intensity (signal M) from probes for methylated CpG sites and the signal intensity (signal U) from probes for non-methylated CpG sites were detected on BeadArray Reader and the methylation rate (mCpG) of CpG sites in ZNF304 was calculated according to the following calculation formula:

$$(\text{mCpG}) = (\text{signal M})/\{(\text{signal M}) + (\text{signal U})\}$$

The threshold was set as "0.4" for the methylation rate (mCpG) of CpG sites of ZNF304, and a specimen having a methylation rate at or higher than the threshold was designated as "methylation positive specimen". Based on the number of methylation positive specimens, methylation positive rate (%) for ZNF304 gene in various tissues was calculated according to the following formula:

$$\text{Methylation positive rate (\%)} = (\text{number of methylation positive specimens/total number of specimens}) \times 100$$

The methylation positive rate of CpG sites of ZNF304 in each tissue is shown in Fig. 6. According to Fig. 6, the CpG sites of ZNF304 showed high methylation positive rate only in cancerous tissues of colon cancer. This indicates that in the promoter region of ZNF304 gene, CpG sites which are not analyzed on the Infinium HumanMethylation27 BeadChip are highly methylated in cancerous tissues of colon cancer.

Reference Example 4: Comparison of methylation data between cancer/tumor tissue specimens derived from several types of cancer/tumor, non-cancerous tissue specimens and normal tissue specimens

(1) Collection of methylation data

In the present Reference Example, methylation data of 7 types of cancer/tumor tissue specimens, 7 types of non-cancerous tissue specimens and 19 types of normal tissue specimens were compared. The number of specimens for the respective tissues is shown in the following tables.

Table 8

| **Cancer/tumor tissue specimens** | |
|---|---|
| **Tissue** | **No. of specimens** |
| **Brain tumor (Brain)** | 114 |
| **Breast cancer (Breast)** | 548 |
| **Lung adenocarcinoma (Lung)** | 220 |
| **Liver cancer (Liver)** | 99 |
| **Colon cancer (colon)** | 324 |
| **Uterine body cancer (Uterus)** | 334 |

(continued)

| Cancer/tumor tissue specimens | |
|---|---|
| **Tissue** | **No. of specimens** |
| **Renal clear cell carcinoma (Kidney)** | 282 |

Table 9

| Non-cancerous tissue | |
|---|---|
| **Tissue** | **No. of specimens** |
| **Brain tumor (Brain)** | 2 |
| **Breast cancer (Breast)** | 98 |
| **Lung adenocarcinoma (Lung)** | 32 |
| **Liver cancer (Liver)** | 19 |
| **Colon cancer (Colon)** | 40 |
| **Uterine body cancer (Uterus)** | 36 |
| **Renal clear cell carcinoma (Kidney)** | 164 |

Table 10

| **Tissue** | RCAST | Literature 1 | Literature 2 | **Total** |
|---|---|---|---|---|
| **Normal brain (Brain)** | 2 | 1 | 0 | 3 |
| **Normal oral cavity (Oral)** | 2 | 0 | 0 | 2 |
| **Normal lung (Lung)** | 0 | 2 | 0 | 2 |
| **Normal colonic mucosa (Colon)** | 2 | 0 | 0 | 2 |
| **Normal liver (Liver)** | 2 | 0 | 0 | 2 |
| **Peripheral blood from healthy subjects (Blood)** | 2 | 2 | 0 | 4 |
| **Normal skeletal muscle (Skeletal)** | 2 | 2 | 0 | 4 |
| **Normal testis (Testis)** | 1 | 0 | 0 | 1 |
| **Normal gastric mucosa (Stomach)** | 0 | 1 | 0 | 1 |
| **Normal pancreas (Pancreas)** | 0 | 2 | 0 | 2 |
| **Normal spleen (Spleen)** | 0 | 2 | 0 | 2 |
| **Normal kidney (Kidney)** | 0 | 0 | 0 | 0 |
| **Normal adrenal gland (Adrenal gland)** | 0 | 2 | 0 | 2 |
| **Normal ureter (Ureter)** | 0 | 2 | 0 | 2 |
| **Normal bladder (Bladder)** | 0 | 2 | 0 | 2 |
| **Normal lymph nodes (Lymph nodes)** | 0 | 2 | 0 | 2 |
| **Normal adipose tissue (Adipose tissue)** | 0 | 2 | 0 | 2 |
| **Normal heart (Heart)** | 0 | 1 | 0 | 1 |
| **Various normal blood cell components** (WB, PBMC, Gran, CD4+, CD8+, CD14+, CD19+, CD56+, Neu, Eos) | 0 | 0 | 60 | 60 |

In Table 10, the methylation data for the specimens indicated in the column "RCAST" were obtained by the present inventors according to Infinium Methylation Assay using the Infinium HumanMethylation450 BeadChip (Illumina). The methylation data for the specimens indicated in the columns "Literature 1" and "Literature 2" were the methylation data published in the following literatures obtained with the Infinium HumanMethylation450 BeadChip. The methylation data in this context are the methylation rate (mCpG) of CpG sites in ZNF304 obtained as described in section (2) in Reference Example 3. The average of the positive rate was plotted for the 18 types of normal tissues excluding the normal blood cell components. For normal blood cell components, the average of 60 specimens of the respective blood cell components from healthy subjects (6 subjects × 10) was plotted.

- Literature 1: Nazor KL et al., Recurrent variations in DNA methylation in human pluripotent stem cells and their differentiated derivatives. Cell Stem Cell 2012; 10 (5): 620-634
- Literature 2: Reinius LE et al., Differential DNA Methylation in Purified Human Blood Cells: Implications for Cell Lineage and Studies on Disease Susceptibility, PLoS One, 7(7) e41361

(2) Comparison of methylation positive rate between cancer/tumor tissue specimens derived from several types of cancer/tumor, non-cancerous tissue specimens and normal tissue specimens

The threshold was set as "0.4" for the methylation rate (mCpG) of genes, and a specimen having a methylation rate at or higher than the threshold was designated as "methylation positive specimen". Based on the number of methylation positive specimens, methylation positive rate (%) for the CpG sites of ZNF304 in various tissues was calculated according to the above formula. The results are shown in Fig. 7. In Fig. 7, "Normal tissues" represent, among the tissues indicated in Table 10, the normal tissues excluding 60 specimens of various normal blood cell components and "Blood cells from healthy subjects" represent the 60 specimens of various normal blood cell components.

Fig. 7 shows that CpG sites of ZNF304 are rarely methylated in other types of cancer or human normal tissues or human normal blood cells and thus are specifically and highly methylated in colon cancer.

Comparative Example 2: Comparison of methylation positive rate between cancer/tumor tissue specimens derived from several types of cancer/tumor, non-cancerous tissue specimens and normal tissue specimens

The methylation positive rate was calculated for GSTP1, CDKN2A and CDH1 genes which are known markers in the similar manner as Reference Example 4 in the respective tissues using the Infinium HumanMethylation450 BeadChip (Illumina). The results are shown in Figs. 8A and 8B.

According to Figs. 8A and 8B, methylation of CDKN2A and MLH1 were detected in other types of cancer than colon cancer, and thus the genes have low specificity towards colon cancer. Thus known markers have issues in terms of performance as diagnostic markers of colon cancer.

Example 3: Methylation analysis of promoter region of ZNF304 gene by MSP

In the present Example, methylation analysis was carried out by MSP for a part of the promoter region in ZNF304 gene other than the one analyzed in Example 2.

(1) Biological samples

In the present Example, the biological samples used were cancerous tissues (5 specimens) of colon cancer. The control samples used were normal colonic mucosa tissues (2 specimens) and non-cancerous colon tissues (2 specimens).

(2) Preparation of measurement samples and control samples

(i) Extraction of genomic DNA

Genomic DNA was extracted from the above tissues with the QIAamp DNA Mini Kit (QIAGEN). Specifically, according to the protocol attached to the kit, each tissue sample was completely lysed in a lysis buffer containing Proteinase K and RNase A and the resulting solution was subjected to absorption on a column. The column was then washed with a washing buffer followed by elution of genomic DNA with 200 μl of sterilized distilled water to obtain purified genomic DNA. The obtained genomic DNA was fragmented with Bioruptor (COSMO BIO).

The control genomic DNA used was genomic DNA of human peripheral blood lymphocytes. In the same manner as in Example 2, the genomic DNA from human peripheral blood lymphocytes was used to prepare non-methylated DNA fragments (0% methylated DNA) and methylated DNA fragments (100% methylated DNA).

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 μl).

(3) MSP

MSP was carried out on the measurement samples and control samples (DNA after bisulfite treatment) obtained in the above section (2). The composition of PCR reagents, primer sets and reaction conditions for PCR in MSP are shown below.

<PCR reagent>

| | |
|---|---|
| DDW (sterilized water) | 16.75 μL |
| 10 × PCR buffer with $MgCl_2$ (Roche) | 2.5 μL |
| 2 mM dNTP mix | 2.5 μL |
| 10 μM sense primer | 1.0 μL |
| 10 μM antisense primer | 1.0 μL |
| Faststart Taq polymerase (Roche) | 0.25 μL |
| Measurement sample or control sample | 1.0 μL |
| Total | 25 μL |

<Primer set>

The primer sets used for MSP are shown in Table 11. The primer set for ZNF304 allows generation of amplification products when DNA in the amplified regions is methylated. A primer set for accuracy control was also used which allows judgment on whether or not the bisulfite treatment had been appropriately carried out. The base sequence (sequence of positive strand) which can be analyzed with the primer set shown in Table 11 in the promoter region of ZNF304 gene is shown in SEQ ID NO: 23.

Table 11

| Primer | | Base sequence of primer | SEQ ID NO: | Annealing temp. (°C) | Cycles |
|---|---|---|---|---|---|
| *ZNF304* | **Sense** | TAATGATAGTTTAGAAATTGGGC | 19 | 54 | 34 |
| | **Antisense** | GAACATAAAAAAAATACGTCACG | 20 | | |
| **Primers for accuracy control** | **Sense** | GGGATATTAAGTGGAGTTATTTTGGTTTTAGTT | 21 | 60 | 40 |
| | **Antisense** | CCCTCCCAACATCCTTCCTAA | 22 | | |

<PCR reaction conditions>

95°C for 6 minutes;
Y cycles of 95°C for 30 seconds, X°C for 30 seconds and 72°C for 30 seconds;
72°C for 7 minutes; and
keep at 16°C.

In the above reaction conditions, "X" and "Y" respectively represent the annealing temperature and the number of cycles as indicated in Table 11.

(4) Analysis of results of MSP

The amplification product from MSP was verified by 2% agarose gel electrophoresis. The results are shown in Fig. 9. In this figure, "0" and "100" under "control" represent the 0% methylation control sample and the 100% methylation control sample, respectively.

According to Fig. 9, bands were detected in all samples of colon cancer tissues while bands derived from methylated CpGs were not detected in normal colonic mucosa tissues or non-cancerous tissues. Accordingly, it was found that CpG sites in the promoter region of the present marker, ZNF304, are highly methylated in colon cancer tissues as detected by methylation analysis by MSP, and thus the marker has high specificity and sensitivity. It is believed that the present marker is a novel marker sufficiently comparable to known colon cancer markers.

Example 4: Evaluation of capability of present markers by mass spectrometry

(1) Biological samples

Cancerous tissues of colon cancer and non-cancerous tissues were collected from 66 patients with colon cancer to obtain 66 sets of tissue samples. In the present Example, the biological samples used were the thus obtained cancerous tissues of colon cancer (66 specimens) and non-cancerous tissues (66 specimens).

(2) Preparation of measurement samples and control samples

(i) Extraction of genomic DNA

In the same manner as in Example 1, genomic DNA was extracted from the above tissues and DNA fragments were obtained. The control genomic DNA used was genomic DNA of human peripheral blood lymphocytes. In the same manner as in Example 1, the genomic DNA from human peripheral blood lymphocytes was used to prepare solutions of non-methylated DNA fragments (0% methylated DNA) and methylated DNA fragments (100% methylated DNA). Further by mixing the 0% methylated DNA solution and the 100% methylated DNA solution at certain proportions, solutions of 25%, 50% and 75% methylated DNA were obtained.

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 $\mu$l).

(iii) Amplification by PCR and IVT

Methylated cytosine and uracil in the DNA fragments after bisulfite treatment were converted respectively to guanine and adenine by PCR and IVT amplification. In the present Example, the primer sets used were those used in Example 1 (see Table 5). The composition of the PCR reaction solution and the reaction conditions of PCR are also the same as those used in Example 1.

The obtained PCR products were dephosphorylated with the MassCLEAVE™ Reagent kit (SEQUENOM) and then subjected to IVT reaction and uracil or thymine specific cleavage reaction. The reagents and reaction conditions used for the treatments are the same as those used in Example 1. The obtained reaction product was purified with Clean Resin (SEQUENOM) to obtain a sample for mass spectrometry. Those samples derived from genomic DNA extracted from the above clinical specimens were designated as measurement samples and samples derived from control genomic DNA were designated as control samples, both of which were used for mass spectrometry described hereinbelow.

(3) Analysis of methylation status by mass spectrometry using MassARRAY®

(i) Preparation of calibration curve

Two mass spectrometric analyses were carried out independently on the control samples obtained as above. On the basis of the obtained analysis results calibration curves were prepared for the respective primer sets and correlation coefficients were calculated. Accordingly it was verified that the primer sets used could amplify both methylated DNA and non-methylated DNA without bias.

(ii) Analysis of measurement samples

The measurement samples obtained as above were subjected to mass spectrometry to obtain peaks of DNA fragments contained in the measurement samples. The obtained peaks were then allocated to the portions of the base sequences of ZNF304 and ZNF264. The methylation score was calculated on the basis of the ratio between the area of the peaks of the fragments containing a methylated CpG site and the area of the peaks of the fragments without the methylated CpG site, both fragments having the same base sequence.

It was verified from the results of quantification of methylation status of non-cancerous colonic mucosa tissues (66 specimens) and cancerous tissues of colon cancer (66 specimens) by MassARRAY analysis that the methylation score was low in non-cancerous colonic mucosa tissues while high in colon cancer. Thus it was demonstrated that the methylation score correlates with colon cancer, similar to the result observed with the Infinium method. Moreover on the basis of the obtained data, boxplots were generated (see Figs. 10A and 10B). In these figures, the vertical axis indicates the corrected methylation score (0 to 1) by MassARRAY. The paired T test was carried out for methylation scores of both markers in cancer tissues and non-cancerous tissues, resulting in demonstration of statistical significance ($p < 0.01$). In addition, cut off value was established by ROC analysis using the corrected methylation scores by MassARRAY and the sensitivity, specificity and AUC were calculated, which are shown in Table 12 and Figs. 11A and 11B.

Table 12

| Marker | ZNF304 | ZNF264 |
|---|---|---|
| **Cut off** | 0.25 | 0.05 |
| **Sensitivity (%)** | 71 | 73 |
| **Specificity (%)** | 94 | 85 |
| AUC | 0.81 | 0.80 |

Preparation of the boxplots and ROC analysis were carried out using a statistical analysis software R 2.15.2 (http://www.r-project.org/) accompanied by a library DiagnosisMed 0.2.3 (http://cran.r-project.org/src/contrib/Archive/DiagnosisMed/) on a computer on which ubuntu® 12.04 had been installed. It is found that the present markers are promising markers having high diagnosis capability because the sensitivity, specificity and AUC were all high in methylation analysis by mass spectrometry using the present markers.

Example 5: Methylation analysis by quantitative methylation specific PCR (quantitative MSP) using blood samples

In the present Example, methylation analysis was carried out by quantitative MSP for CpG sites in promoter regions of ZNF304 and ZNF264 using peripheral blood as a biological sample.

(1) Biological samples

In the present Example, the biological samples used were peripheral blood collected from patients with colon cancer (6 specimens). The control samples used were peripheral blood from healthy subjects (3 specimens).

(2) Preparation of measurement samples and control samples

(i) Extraction of genomic DNA

Plasma was obtained from each of the above peripheral blood (2 ml) according to the standard method. Genomic DNA was extracted from the resulting plasma with the QIAamp Circulating Nucleic Acid Kit (QIAGEN).

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 μl).

(3) Quantitative MSP

Quantitative MSP was carried out with the measurement samples obtained in the above section (2). The composition of PCR reagents, primer sets and reaction conditions for PCR in quantitative MSP are shown below.

<PCR reagent>

| | |
|---|---|
| DW (sterilized water) | (A) μL |
| 2 × FastStart Universal Probe Master Mix (Roche) | 2.5 μL |
| 10 μM TaqMan® probe | (B) μL |
| 10 μM sense primer | 1.0 μL |
| 10 μM antisense primer | 1.0 μL |
| Measurement sample | 1.0 μL |
| Total | 25 μL |

In the above composition, (A) and (B) respectively represent the volume (μl) indicated in Table 13.

Table 13

| | (A) | (B) |
|---|---|---|
| ZNF264 | 5.4 | 0.6 |
| ZNF304 | 5.5 | 0.5 |

<Primer set>

The primer sets used for the quantitative MSP and standard oligoDNAs used for generation of standard curves are shown in Table 14.

Table 14

| | | Base sequence | SEQ ID NO: |
|---|---|---|---|
| ZNF264 | Sense primer | ATTTATTTCGGAAATGGTTTTAGG | 24 |
| | Antisense primer | ACTCTACGACCCAAAAAATAACGTA | 25 |
| | TaqMan® probe | TGGTAAGGAAGCGGTAATCGCGAA | 26 |
| | Standard oligoDNA | ATTTATTTCGGAAATCGTTTTAGCGTTGGTAA GGAAGCGGTAATCGCGAAGTTATCGTTTATTT ATTTGGGTTCGGTTCGTTAGTATCGTTGTCGT TATTACGTTATTTTTTGGGTCGTAGAGT | 27 |
| ZNF304 | Sense primer | TTGTATTTAATTAGGTAACGAGCGT | 28 |
| | Antisense primer | TTTAAAAACAACTATAACCTCCGAA | 29 |
| | TaqMan® probe | TCGGATGTTTGTTAAGAGGCGTCG | 30 |
| | Standard oligoDNA | TTGTATTTAATTAGGTAACGAGCGTTTTTCGG GTTTTTTCGGATGTTTGTTAAGAGGCGTCGTG TAGGGAGGGGGAGAAAAGATTGGGAAAACGA TATTTCGGAGGTTATAGTTGTTTTTAAA | 31 |

<PCR reaction conditions >

95°C for 10 minutes;

50 cycles of 95°C for 30 seconds, X°C for 30 seconds and 72°C for 30 seconds; and

keep at 16°C.

In the above reaction conditions, "X" represents annealing temperature which is 60°C for ZNF264 and 56°C for ZNF304.

(4) Qualitative MSP

Qualitative MSP was carried out for the measurement samples obtained in the above section (2) with the primer set for accuracy control. The composition of PCR reagents and reaction conditions for PCR used in qualitative MSP are shown below. The base sequences of the primer set for accuracy control are previously shown in Table 11.

<PCR reagent>

| | |
|---|---|
| DW (sterilized water) | 10.88 μL |
| 10 × PCR buffer with $MgCl_2$ (Roche) | 1.5 μL |
| 10 mM dNTP mix | 0.3 μL |
| 10 μM sense primer | 0.6 μL |
| 10 μM antisense primer | 0.6 μL |
| Faststart Taq polymerase (Roche) | 0.12 μL |
| Measurement sample | 1.0 μL |
| Total | 15 μL |

<PCR reaction conditions >

95°C for 6 minutes;
50 cycles of 95°C for 30 seconds, 60°C for 30 seconds and 72°C for 30 seconds;
72°C for 7 minutes; and
keep at 16°C.

(5) Analysis of results of MSP

A standard curve was generated using the result with standard oligoDNA having known copy number. Based on the standard curve, the DNA copy number for each sample was calculated from the amplification data obtained by quantitative MSP as in the above section (3). Figs. 12A and 12B show the graphs of the calculated DNA copy numbers. For the internal standard (accuracy control) obtained by qualitative MSP as in the above section (4), the band intensity of amplification products after 2% agarose gel electrophoresis of the reaction solution is shown in a graph (see Fig. 12C).

By analyzing methylation status of plasma from healthy subjects (3 specimens) and plasma from colon cancer patients (6 specimens) by quantitative MSP analysis, DNA amplification was not detected for plasma from healthy subjects while DNA amplification was detected for ZNF304 and ZNF264 in 3 samples among 6 samples of plasma from colon cancer patients. Bands were detected in all samples by qualitative PCR with the primer set for accuracy control. This indicates that the bisulfite treatment of the samples had been appropriately carried out. Accordingly it is found that the present markers tend to be highly methylated in colon cancer patients and methylation thereof is not detected in healthy subjects even when the biological sample used is peripheral blood. Therefore it is suggested that the present markers are highly specific for colon cancer and are useful for determining presence or absence of cancer cells derived from colon cancer in blood.

Example 6: Methylation analysis by MSP using blood samples

In the present Example, methylation analysis was carried out by MSP for CpG sites in promoter region of ZNF304 gene analyzed by the Infinium HumanMethylation450 BeadChip (Illumina) using peripheral blood as a biological sample.

(1) Biological samples

In the present Example, the biological samples used were peripheral blood collected from patients with colon cancer (6 specimens). The control samples used were peripheral blood from healthy subjects (3 specimens).

(2) Preparation of measurement samples and control samples

(i) Extraction of genomic DNA

Plasma was obtained from each of the above peripheral blood (2 ml) according to the standard method. Genomic DNA was extracted from the resulting plasma with the QIAamp Circulating Nucleic Acid Kit (QIAGEN).

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 μl).

(3) Quantitative MSP

Qualitative MSP was carried out with the measurement samples obtained in the above section (2). The composition of PCR reagents are the same as those used for qualitative MSP in Example 5. The primer set used is shown in Table 15. The base sequences of the primer set for accuracy control are previously shown in Table 11.

Table 15

| | | Base sequence | SEQ ID NO: |
|---|---|---|---|
| ZNF304 | Sense primer | TAATGATAGTTTAGAAATTGGGC | 32 |
| | Antisense primer | GAACATAAAAAAAATACGTCACG | 33 |

The PCR reaction conditions are as follows:

<PCR reaction conditions>

95°C for 6 minutes;
50 cycles of 95°C for 30 seconds, X°C for 30 seconds and 72°C for 30 seconds;
72°C for 7 minutes; and
keep at 16°C.

In the above reaction conditions, "X" represents annealing temperature which is 54°C for ZNF304 and 60°C for the primer set for accuracy control.

(4) Analysis of results of MSP

The amplification product from MSP was verified by 2% agarose gel electrophoresis. The band intensities of amplification products were represented in graphs (only the graph for ZNF304 is shown in Fig. 13). The bars in Fig. 13 indicated under peripheral blood of healthy subjects correspond to the background, indicating absence of detection of any band. According to Fig. 13, it is found that PCR for ZNF304 allowed detection of no band in peripheral blood from healthy subjects and detection of bands in 5 samples among 6 samples of peripheral blood from colon cancer patients. Bands were detected in all samples by qualitative PCR with the primer set for accuracy control. This indicates that the bisulfite treatment of the samples had been appropriately carried out. Accordingly it is found that the present markers tend to be highly methylated in colon cancer patients and methylation thereof is not detected in healthy subjects even when the biological sample used is peripheral blood. Therefore it is suggested that the present markers are highly specific for colon cancer and are useful for determining presence or absence of cancer cells derived from colon cancer in blood samples.

Reference Signs List

1 Determination device
2 Measurement device
3 Computer system
3a Computer main body
3b Input device
3c Display

## Claims

1. A method for obtaining information on the presence or absence of a cancer cell derived from colon cancer in a biological sample collected from a subject comprising the steps of:

   preparing a DNA sample from the biological sample collected from the subject;
   analyzing methylation status of a CpG site in a promoter region of at least one gene selected from ZNF304 and ZNF264 in the DNA sample obtained from the preparation step; and
   obtaining information on the presence or absence of a cancer cell derived from colon cancer in the biological sample collected from the subject based on an analysis result obtained from the analyzing step.

2. The method according to claim 1, wherein the analyzing step is the step of analyzing presence or absence of methylation of at least one CpG site.

3. The method according to claim 2, wherein the step of obtaining information is the step of obtaining information indicating that the biological sample contains a cancer cell derived from colon cancer when the analysis result shows that there is a methylated CpG site.

4. The method according to claim 1, wherein the analyzing step is the step of analyzing methylation frequency.

5. The method according to claim 4, wherein the step of obtaining information is the step of obtaining information indicating that the biological sample contains a cancer cell derived from colon cancer when the methylation frequency obtained from the analyzing step is higher than a predetermined threshold.

6. Use of a kit in a method for obtaining information on the presence or absence of a cancer cell derived from colon cancer in a biological sample collected from a subject according to claim 1, said kit comprising a primer set for analysis of methylation of at least one CpG site selected from CpG sites located in promoter regions of ZNF304 and ZNF264 genes.

7. Use according to claim 6, wherein the primer set is a primer set for analyzing methylation status of the CpG site by at least one method selected from mass spectrometry and methylation specific PCR method.

8. Use according to claim 7, wherein the primer set is at least one selected from:

   a primer set of primers respectively having base sequences SEQ ID NOs: 3 and 4;
   a primer set of primers respectively having base sequences SEQ ID NOs: 5 and 6;
   a primer set of primers respectively having base sequences SEQ ID NOs: 9 and 10;
   a primer set of primers respectively having base sequences SEQ ID NOs: 11 and 12; and
   a primer set of primers respectively having base sequences SEQ ID NOs: 19 and 20.

## Patentansprüche

1. Verfahren zur Gewinnung von Informationen über die Anwesenheit oder Abwesenheit einer aus Darmkrebs stammenden Krebszelle in einer aus einem Individuum entnommenen biologischen Probe, umfassend die Schritte:

Aufbereiten einer DNA-Probe aus der aus dem Individuum entnommenen biologischen Probe;
Analysieren des Methylierungszustands einer CpG-Stelle in einer Promoterregion von wenigstens einem aus ZNF304 und ZNF264 ausgewählten Gen in der aus dem Aufbereitungsschritt gewonnenen DNA-Probe; und
Gewinnen von Informationen über die Anwesenheit oder Abwesenheit einer aus Darmkrebs stammenden Krebszelle in der aus dem Individuum entnommenen biologischen Probe basierend auf einem aus dem Analyseschritt gewonnenen Analyseergebnis.

**2.** Verfahren nach Anspruch 1, wobei der Analyseschritt der Schritt der Analyse der Anwesenheit oder Abwesenheit von Methylierung von wenigstens einer CpG-Stelle ist.

**3.** Verfahren nach Anspruch 2, wobei
der Schritt zur Gewinnung von Informationen der Schritt zur Gewinnung von Informationen ist, die darauf hinweisen, dass die biologische Probe eine aus Darmkrebs stammende Krebszelle enthält, wenn das Analyseergebnis zeigt, dass es eine methylierte CpG-Stelle gibt.

**4.** Verfahren nach Anspruch 1, wobei der Analyseschritt der Schritt der Analyse der Methylierungshäufigkeit ist.

**5.** Verfahren nach Anspruch 4, wobei
der Schritt zur Gewinnung von Informationen der Schritt zur Gewinnung von Informationen ist, die darauf hinweisen, dass die biologische Probe eine aus Darmkrebs stammende Krebszelle enthält, wenn die aus dem Analyseschritt gewonnene Methylierungshäufigkeit höher als ein zuvor festgelegter Schwellenwert ist.

**6.** Verwendung eines Kits in einem Verfahren zur Gewinnung von Informationen über die Anwesenheit oder Abwesenheit einer aus Darmkrebs stammenden Krebszelle in einer aus einem Individuum entnommenen biologischen Probe nach Anspruch 1, wobei das Kit einen Primersatz zur Analyse von Methylierung von wenigstens einer aus CpG-Stellen, die sich in Promoterregionen von ZNF304- und ZNF264-Genen befinden, ausgewählten CpG-Stelle enthält.

**7.** Verwendung nach Anspruch 6, wobei der Primersatz ein Primersatz zur Analyse des Methylierungszustands der CpG-Stelle durch wenigstens ein aus Massenspektrometrie und methylierungsspezifischem PCR-Verfahren ausgewähltes Verfahren ist.

**8.** Verfahren nach Anspruch 7, wobei der Primersatz wenigstens einer ist, der ausgewählt ist aus:

einem Primersatz von Primern, die jeweils die Basensequenzen SEQ ID NO: 3 und 4 aufweisen;
einem Primersatz von Primern, die jeweils die Basensequenzen SEQ ID NO: 5 und 6 aufweisen;
einem Primersatz von Primern, die jeweils die Basensequenzen SEQ ID NO: 9 und 10 aufweisen,
einem Primersatz von Primern, die jeweils die Basensequenzen SEQ ID NO: 11 und 12 aufweisen; und
einem Primersatz von Primern, die jeweils die Basensequenzen SEQ ID NO: 19 und 20 aufweisen.

**Revendications**

**1.** Procédé d'obtention d'informations sur la présence ou l'absence d'une cellule cancéreuse dérivée d'un cancer du côlon dans un échantillon biologique collecté à partir d'un sujet comprenant les étapes de :

préparation d'un échantillon d'ADN à partir de l'échantillon biologique collecté à partir du sujet ;
analyse de l'état de méthylation d'un site CpG dans une région de promoteur d'au moins un gène choisi parmi ZNF304 et ZNF264 dans l'échantillon d'ADN obtenu dans l'étape de préparation ; et
obtention d'informations sur la présence ou l'absence d'une cellule cancéreuse dérivée d'un cancer du côlon dans l'échantillon biologique collecté à partir du sujet sur la base d'un résultat d'analyse obtenu à partir de l'étape d'analyse.

**2.** Procédé selon la revendication 1, dans lequel l'étape d'analyse est l'étape d'analyse de la présence ou l'absence de méthylation d'au moins un site CpG.

3. Procédé selon la revendication 2, dans lequel l'étape d'obtention d'informations est l'étape d'obtention d'informations indiquant que l'échantillon biologique contient une cellule cancéreuse dérivée d'un cancer du côlon lorsque le résultat d'analyse montre qu'il existe un site CpG méthylé.

4. Procédé selon la revendication 1, dans lequel l'étape d'analyse est l'étape d'analyse de fréquence de méthylation.

5. Procédé selon la revendication 4, dans lequel l'étape d'obtention d'informations est l'étape d'obtention d'informations indiquant que l'échantillon biologique contient une cellule cancéreuse dérivée d'un cancer du côlon lorsque la fréquence de méthylation obtenue dans l'étape d'analyse est supérieure à un seuil prédéterminé.

6. Utilisation d'une trousse dans un procédé d'obtention d'informations sur la présence ou l'absence d'une cellule cancéreuse dérivée d'un cancer du côlon dans un échantillon biologique collecté à partir d'un sujet selon la revendication 1, ladite trousse comprenant un ensemble d'amorces pour l'analyse de méthylation d'au moins un site CpG choisi parmi des sites CpG situés dans des régions de promoteur des gènes ZNF304 et ZNF264.

7. Utilisation selon la revendication 6, dans lequel l'ensemble d'amorces est un ensemble d'amorces pour analyser l'état de méthylation du site CpG par au moins un procédé choisi parmi un procédé de spectrométrie de masse et de PCR spécifique pour la méthylation.

8. Utilisation selon la revendication 7, dans laquelle l'ensemble d'amorces est au moins l'un choisi parmi :

un ensemble d'amorces comprenant des amorces ayant respectivement les séquences de base SEQ ID NO: 3 et 4 ;
un ensemble d'amorces comprenant des amorces ayant respectivement les séquences de base SEQ ID NO: 5 et 6 ;
un ensemble d'amorces comprenant des amorces ayant respectivement les séquences de base SEQ ID NO: 9 et 10 ;
un ensemble d'amorces comprenant des amorces ayant respectivement les séquences de base SEQ ID NO: 11 et 12 ; et
un ensemble d'amorces comprenant des amorces ayant respectivement les séquences de base SEQ ID NO: 19 et 20.

Fig. 1A

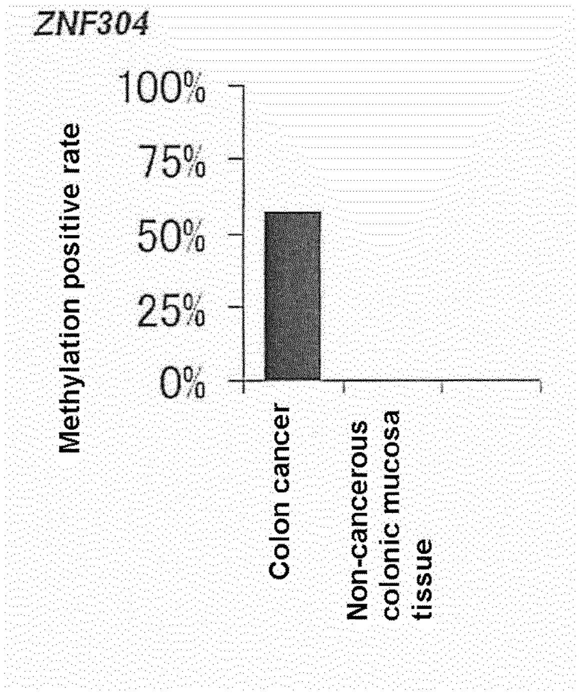
ZNF304
Methylation positive rate
100%
75%
50%
25%
0%
Colon cancer
Non-cancerous colonic mucosa tissue

Fig.1B

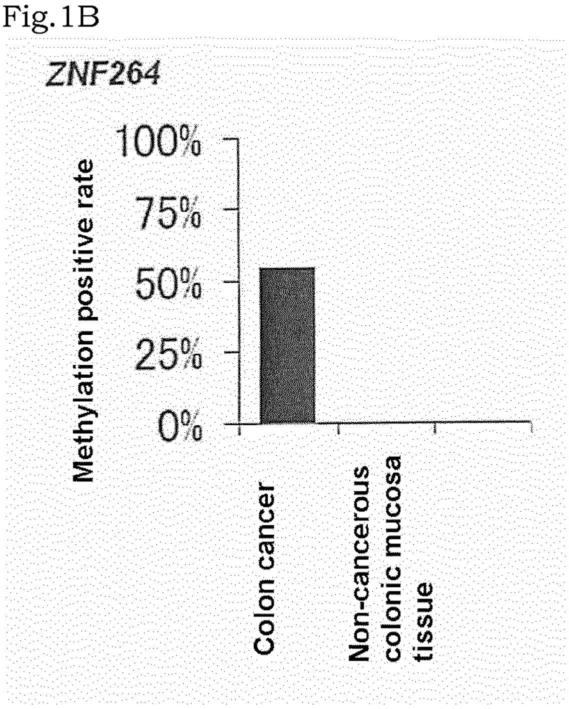
ZNF264
Methylation positive rate
100%
75%
50%
25%
0%
Colon cancer
Non-cancerous colonic mucosa tissue

Fig. 2A

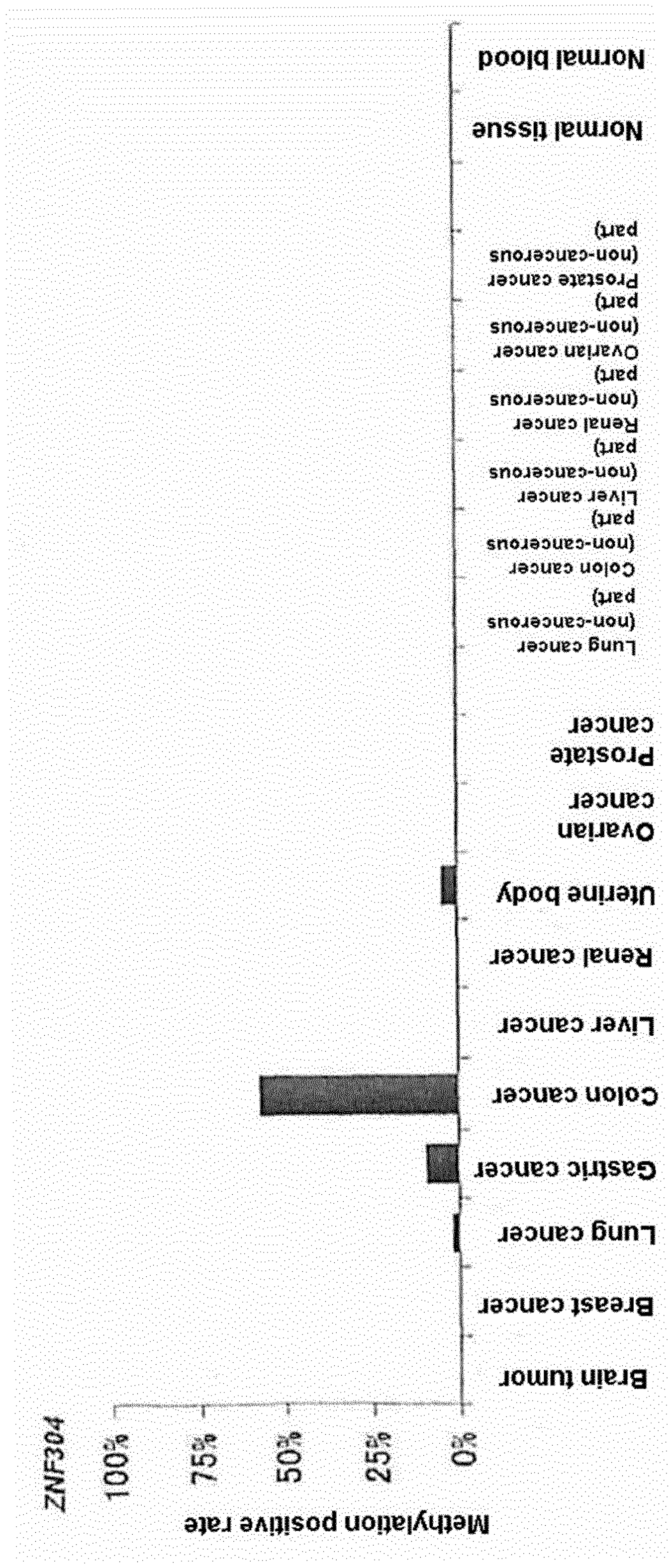
ZNF304
Methylation positive rate
100%
75%
50%
25%
0%
Brain tumor
Breast cancer
Lung cancer
Gastric cancer
Colon cancer
Liver cancer
Renal cancer
Uterine body
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

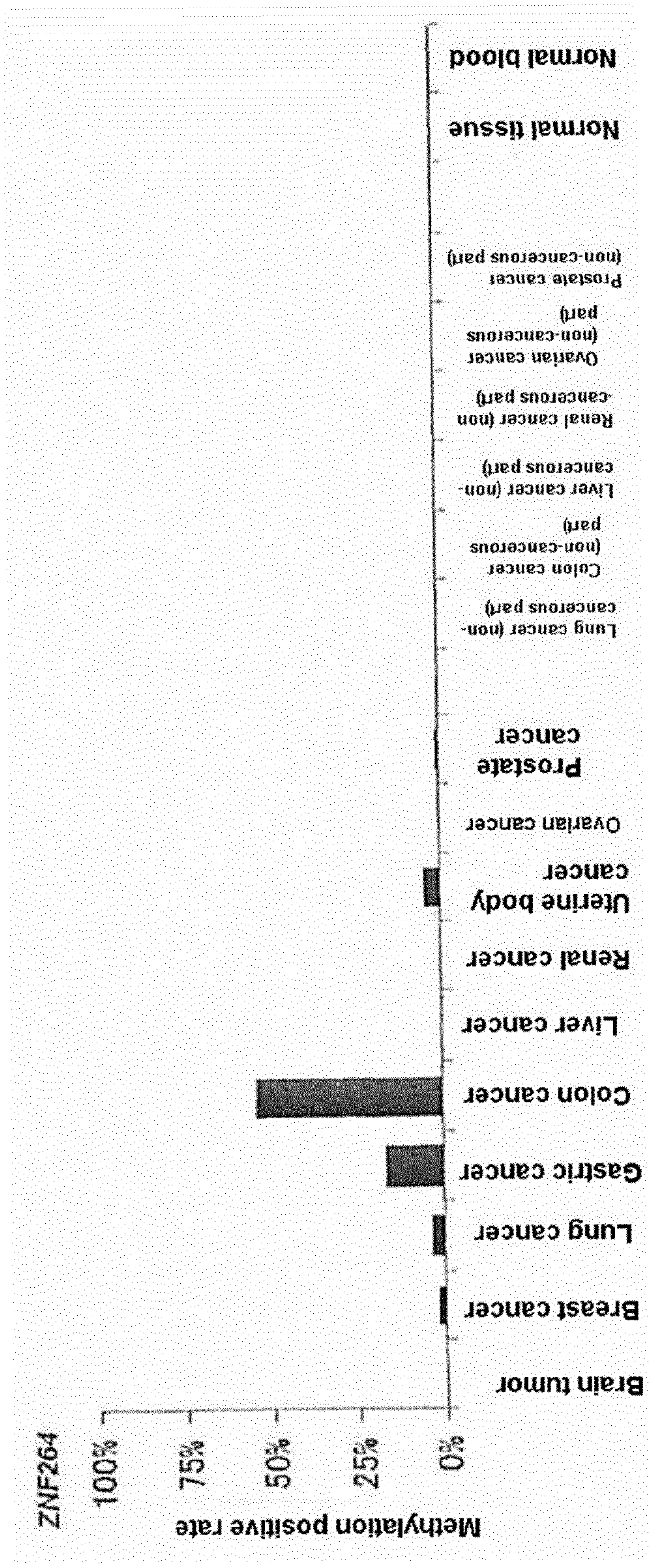
ZNF264
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

Fig. 2B

Fig. 3A

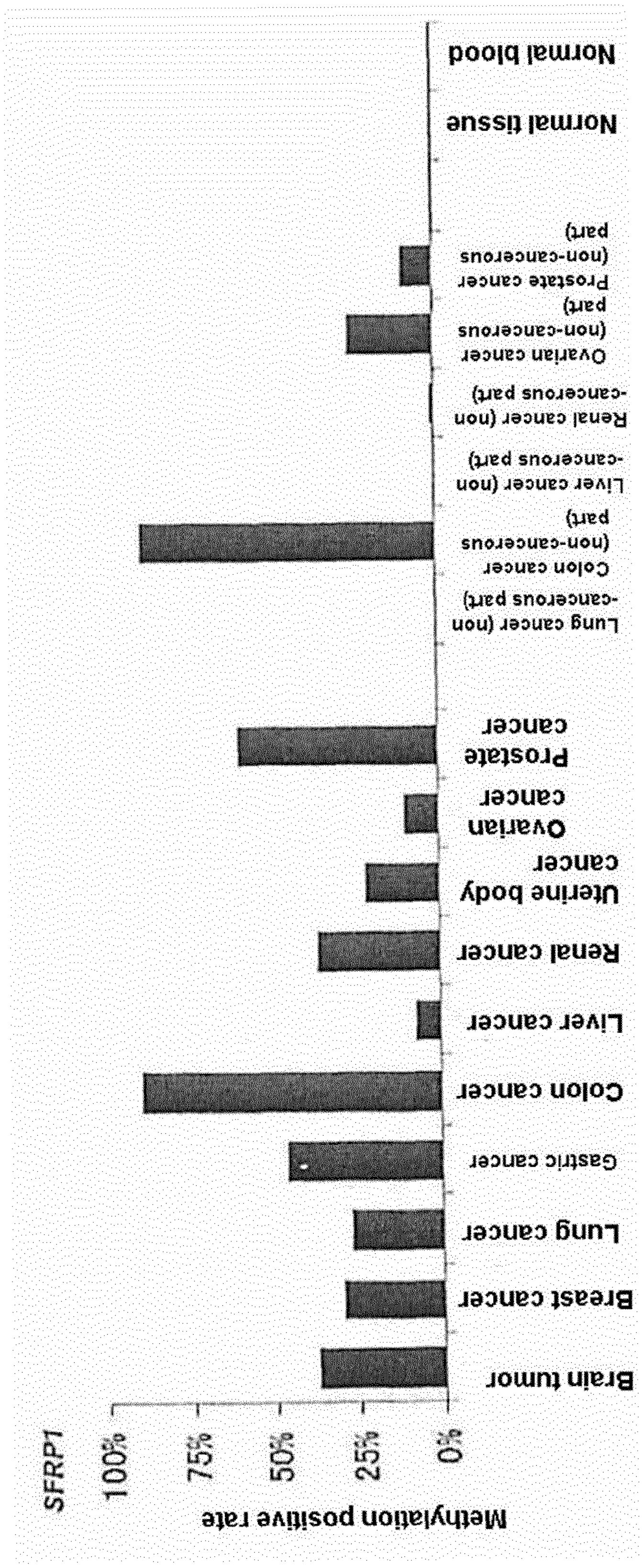
SFRP1
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

Fig. 3B

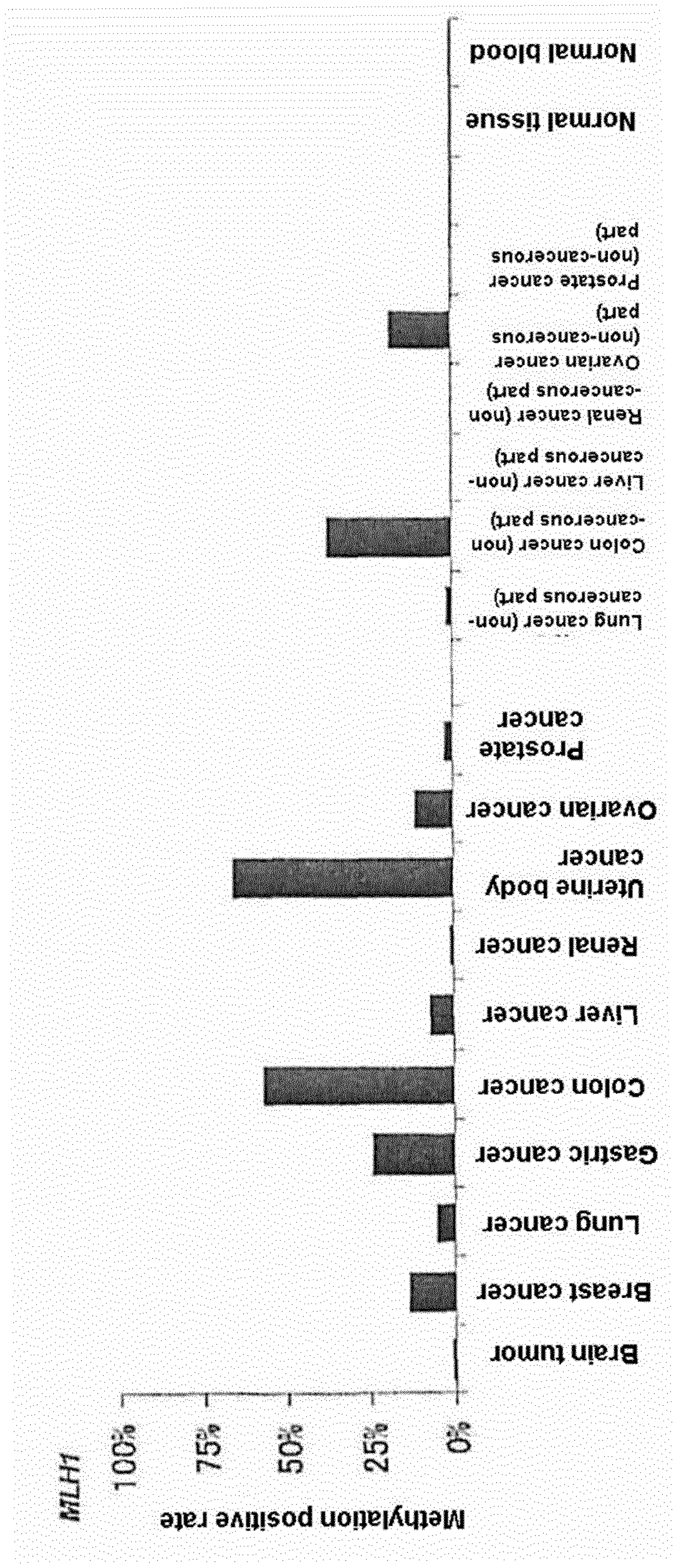
MLH1
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

Fig. 3C

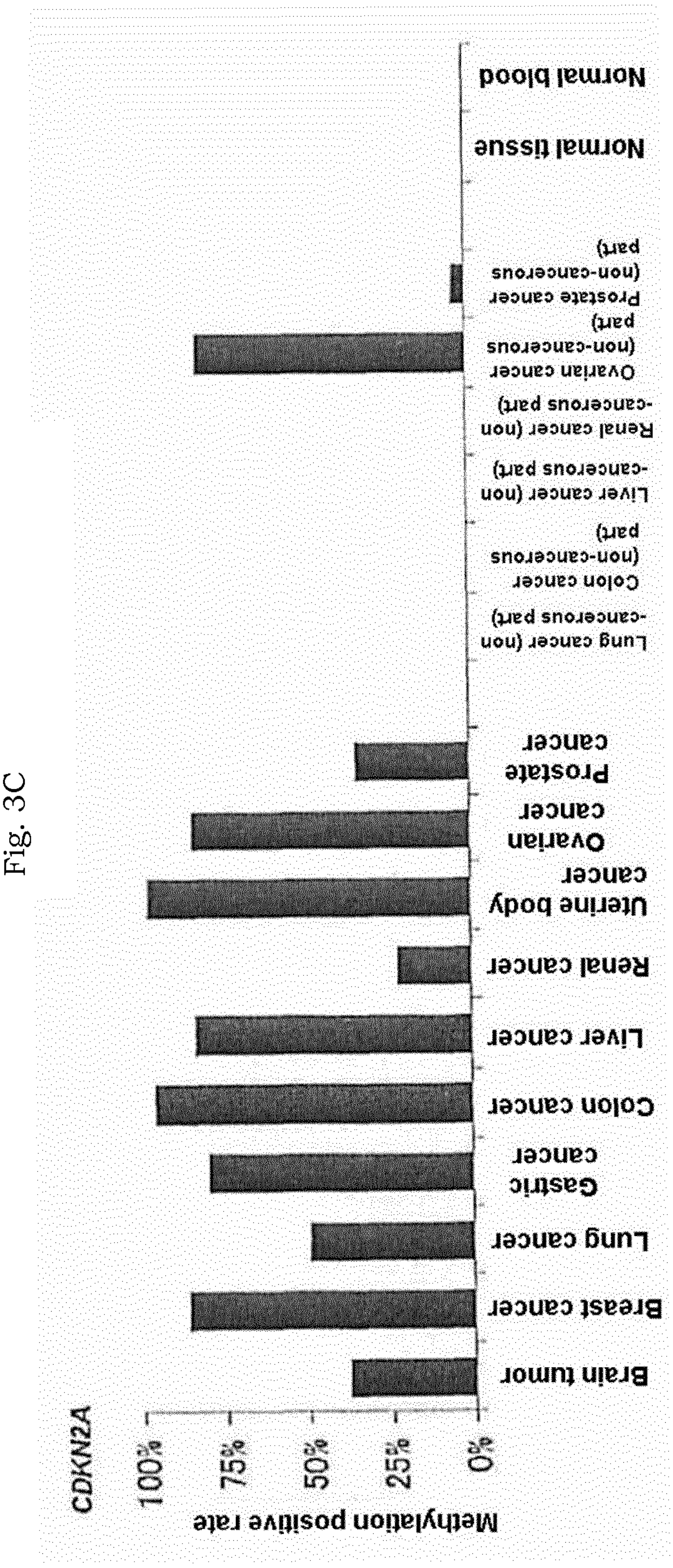
CDKN2A
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

Fig. 4A

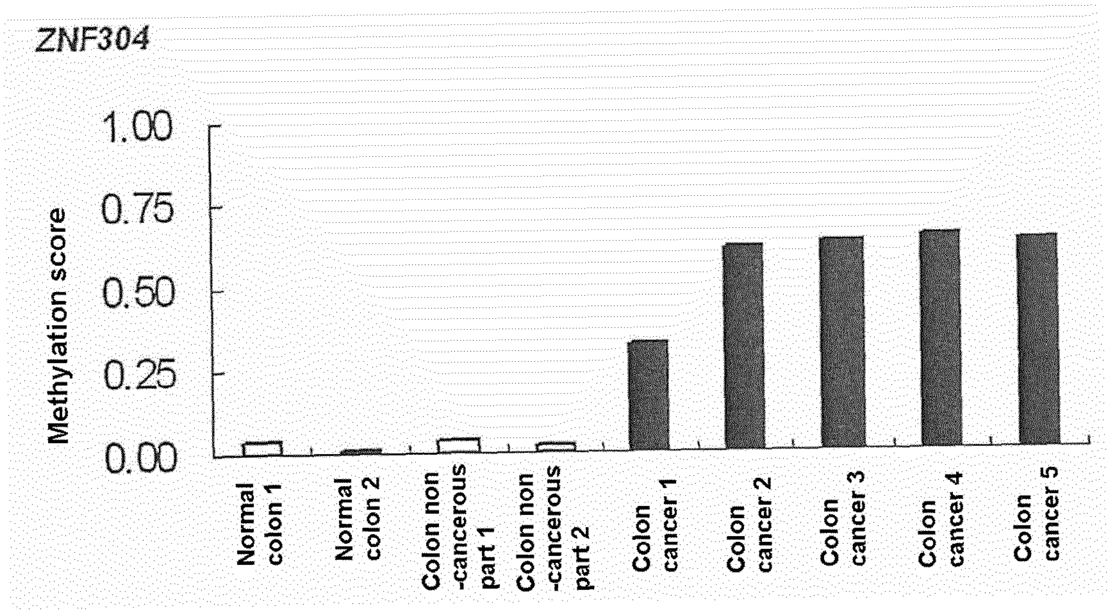
ZNF304
Methylation score
1.00
0.75
0.50
0.25
0.00
Normal colon 1
Normal colon 2
Colon non -cancerous part 1
Colon non -cancerous part 2
Colon cancer 1
Colon cancer 2
Colon cancer 3
Colon cancer 4
Colon cancer 5

Fig. 4B

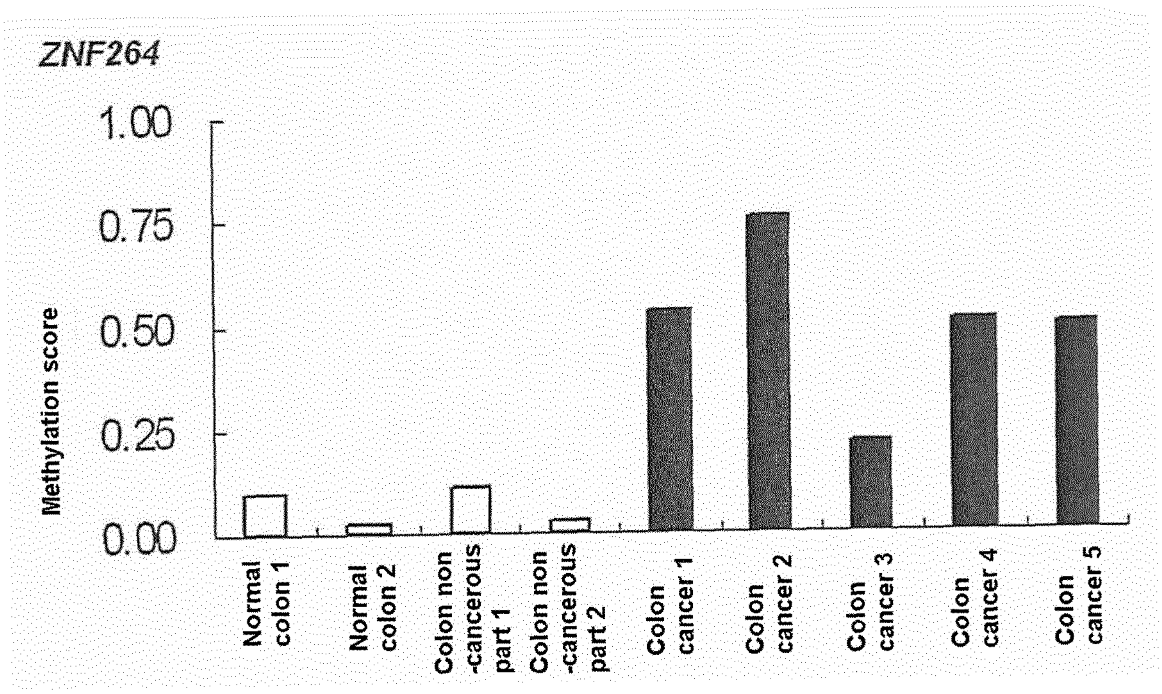
ZNF264
Methylation score
1.00
0.75
0.50
0.25
0.00
Normal colon 1
Normal colon 2
Colon non -cancerous part 1
Colon non -cancerous part 2
Colon cancer 1
Colon cancer 2
Colon cancer 3
Colon cancer 4
Colon cancer 5

Fig. 5

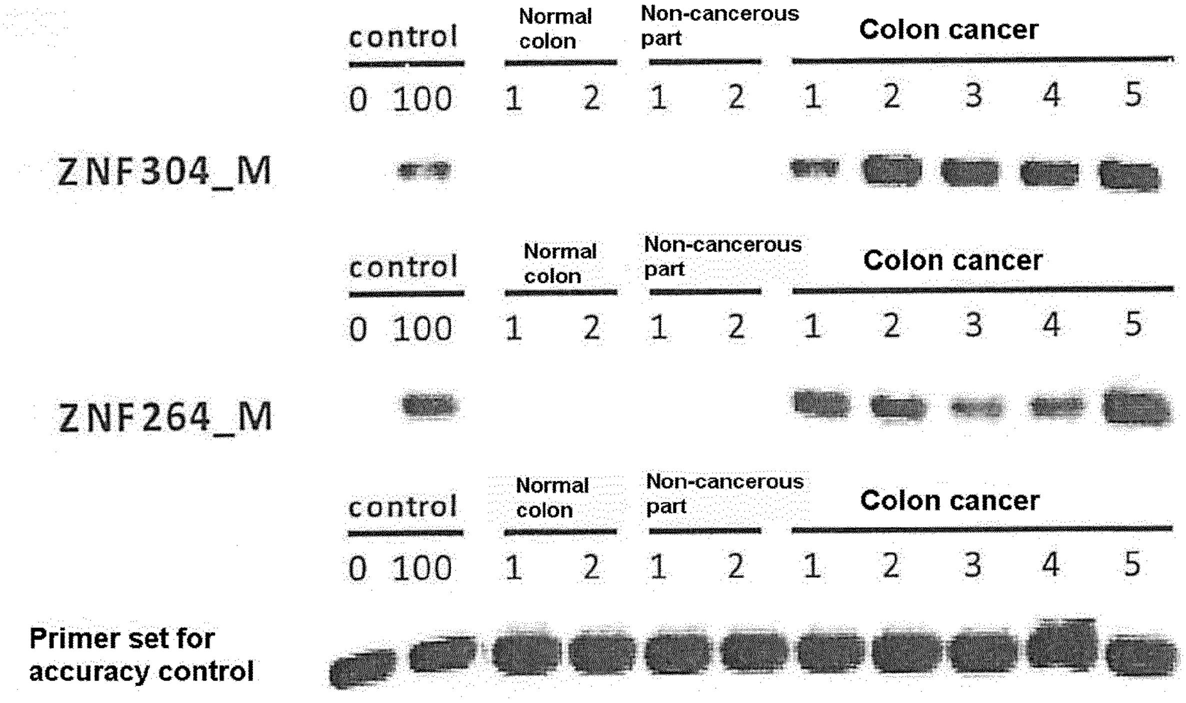
control
Normal colon
Non-cancerous part
Colon cancer
0 100 1 2 1 2 1 2 3 4 5
ZNF304_M
control
Normal colon
Non-cancerous part
Colon cancer
0 100 1 2 1 2 1 2 3 4 5
ZNF264_M
control
Normal colon
Non-cancerous part
Colon cancer
0 100 1 2 1 2 1 2 3 4 5
Primer set for accuracy control

Fig. 6

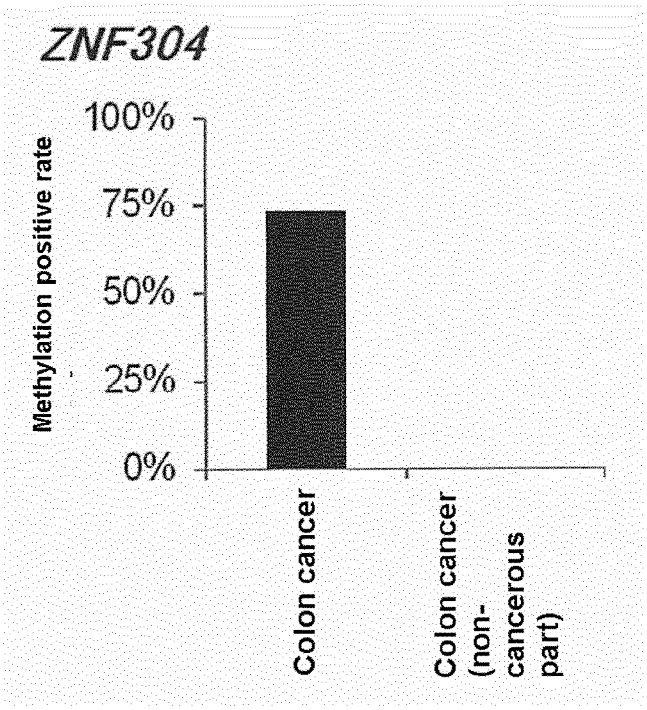
ZNF304
Methylation positive rate
100%
75%
50%
25%
0%
Colon cancer
Colon cancer (non-cancerous part)

Fig. 7

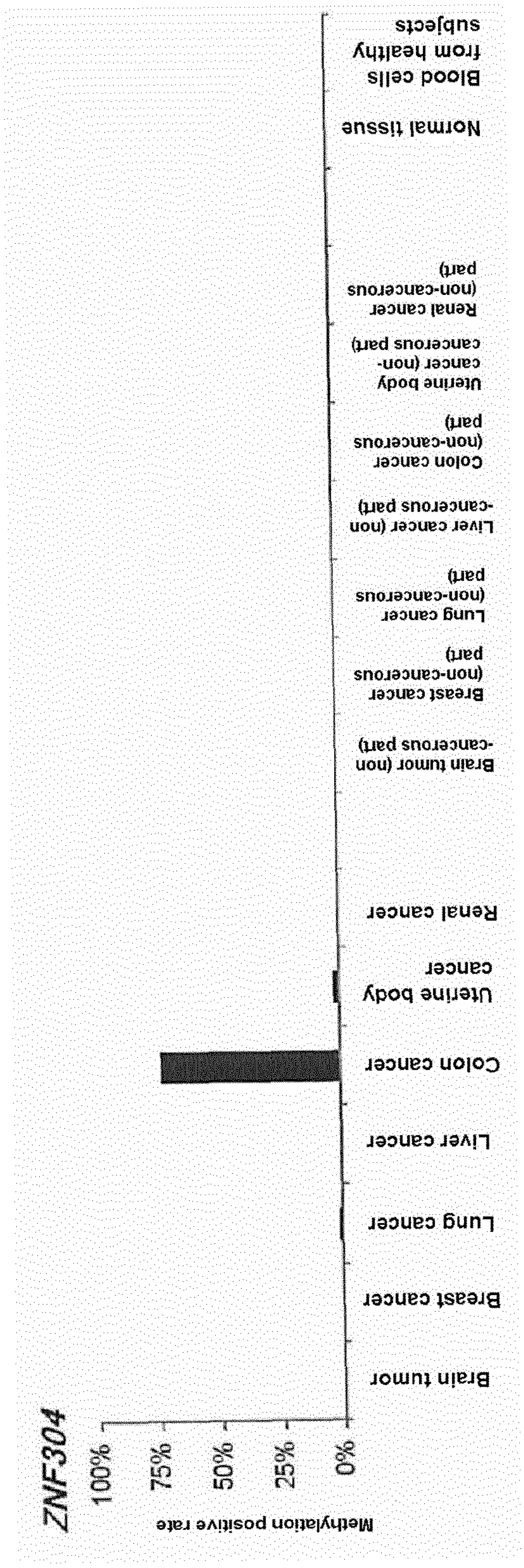
ZNF304
Methylation positive rate
100%
75%
50%
25%
0%
Brain tumor
Breast cancer
Lung cancer
Liver cancer
Colon cancer
Uterine body cancer
Renal cancer
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissue
Blood cells from healthy subjects

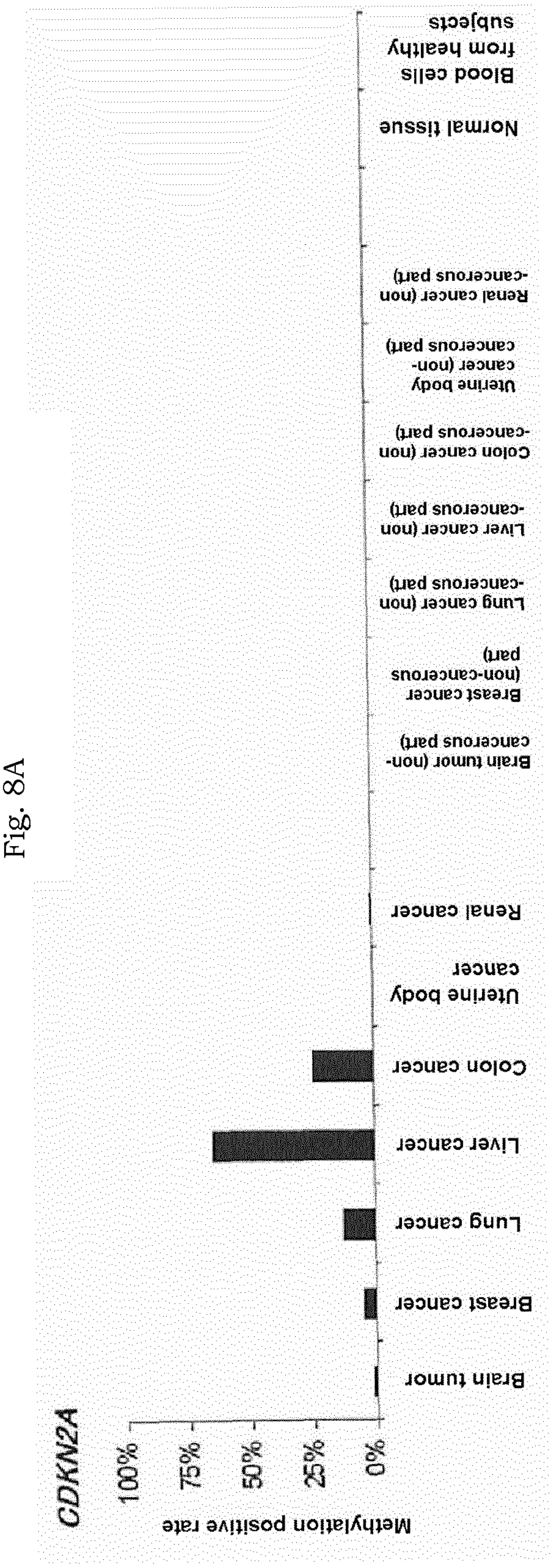
CDKN2A
Methylation positive rate
100%
75%
50%
25%
0%
Brain tumor
Breast cancer
Lung cancer
Liver cancer
Colon cancer
Uterine body cancer
Renal cancer
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissue
Blood cells from healthy subjects

Fig. 8A

Fig. 8B

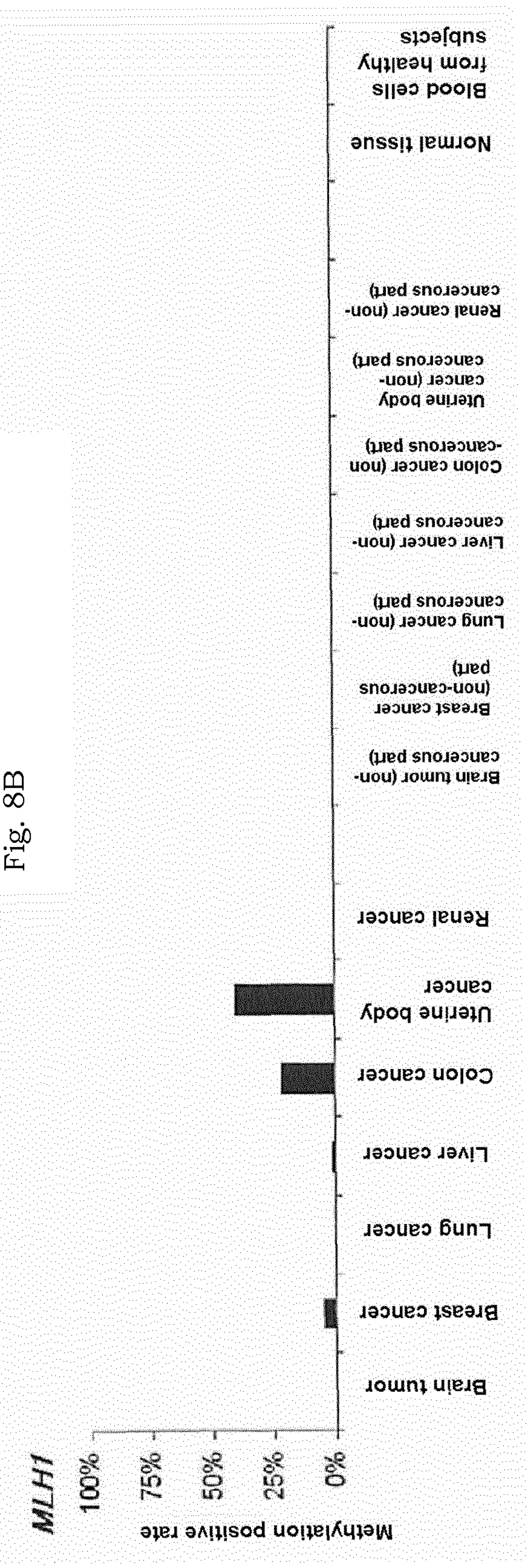
MLH1
Methylation positive rate
100%
75%
50%
25%
0%
Brain tumor
Breast cancer
Lung cancer
Liver cancer
Colon cancer
Uterine body cancer
Renal cancer
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissue
Blood cells from healthy subjects

Fig. 9

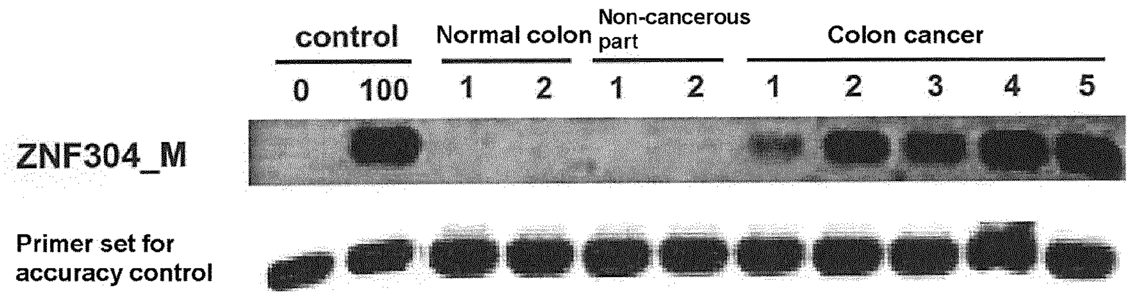
control
Normal colon
Non-cancerous part
Colon cancer
0
100
1
2
1
2
1
2
3
4
5
ZNF304_M
Primer set for accuracy control

Fig. 10A

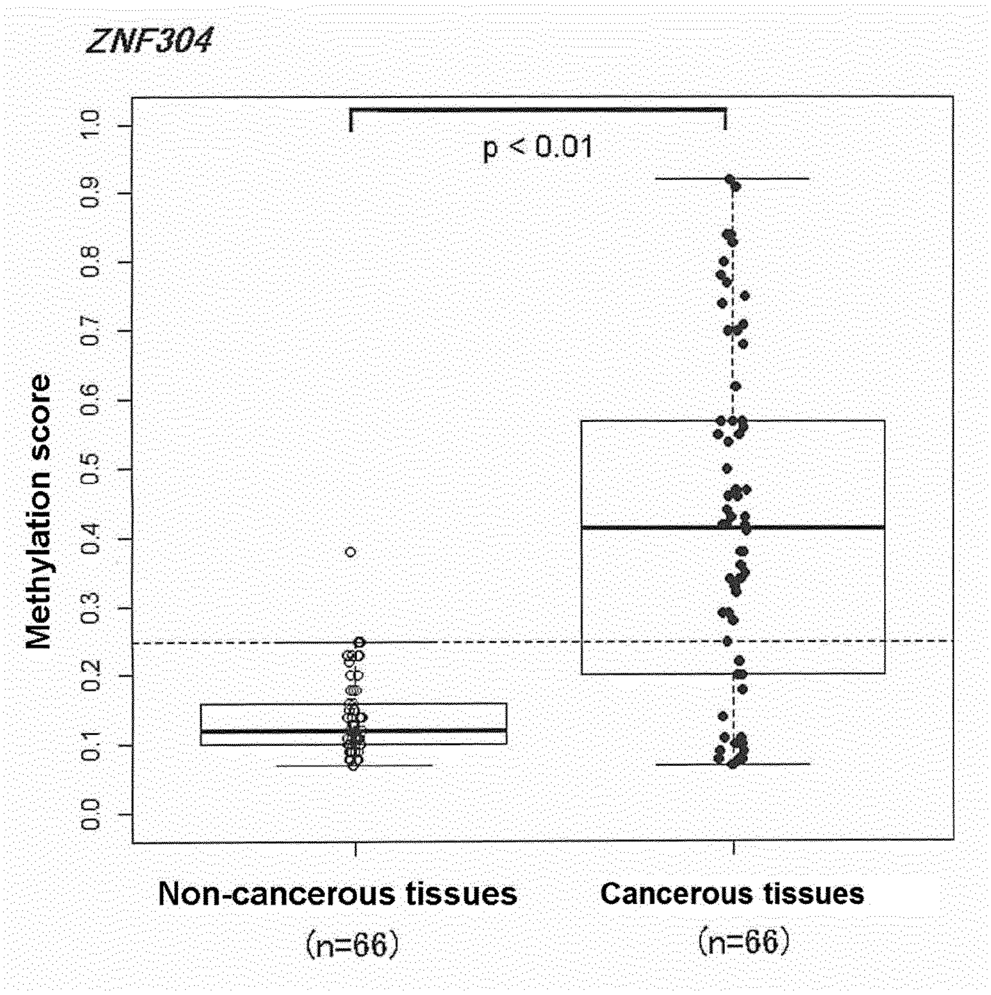
ZNF304
p < 0.01
Methylation score
0.0
0.1
0.2
0.3
0.4
0.5
0.6
0.7
0.8
0.9
1.0
Non-cancerous tissues
(n=66)
Cancerous tissues
(n=66)

Fig. 10B

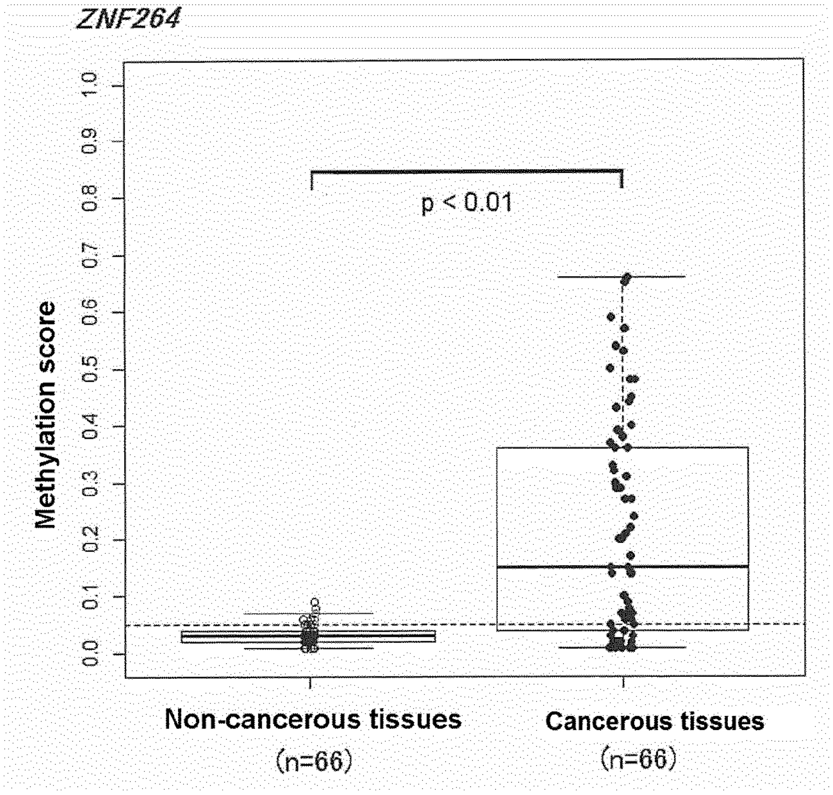


Fig. 11A

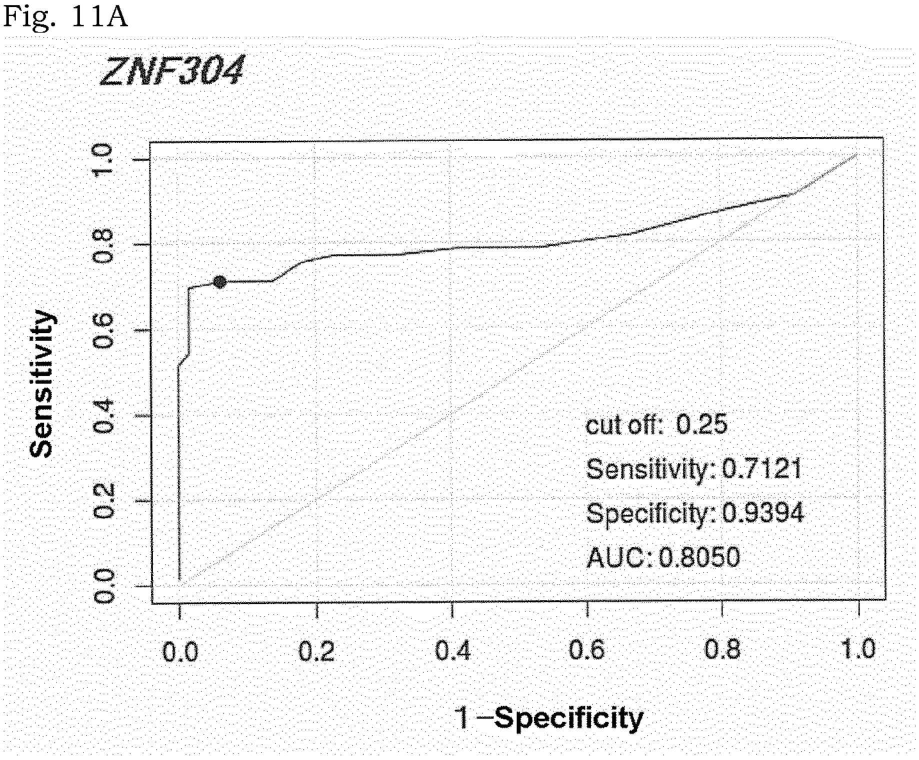

Fig. 11B

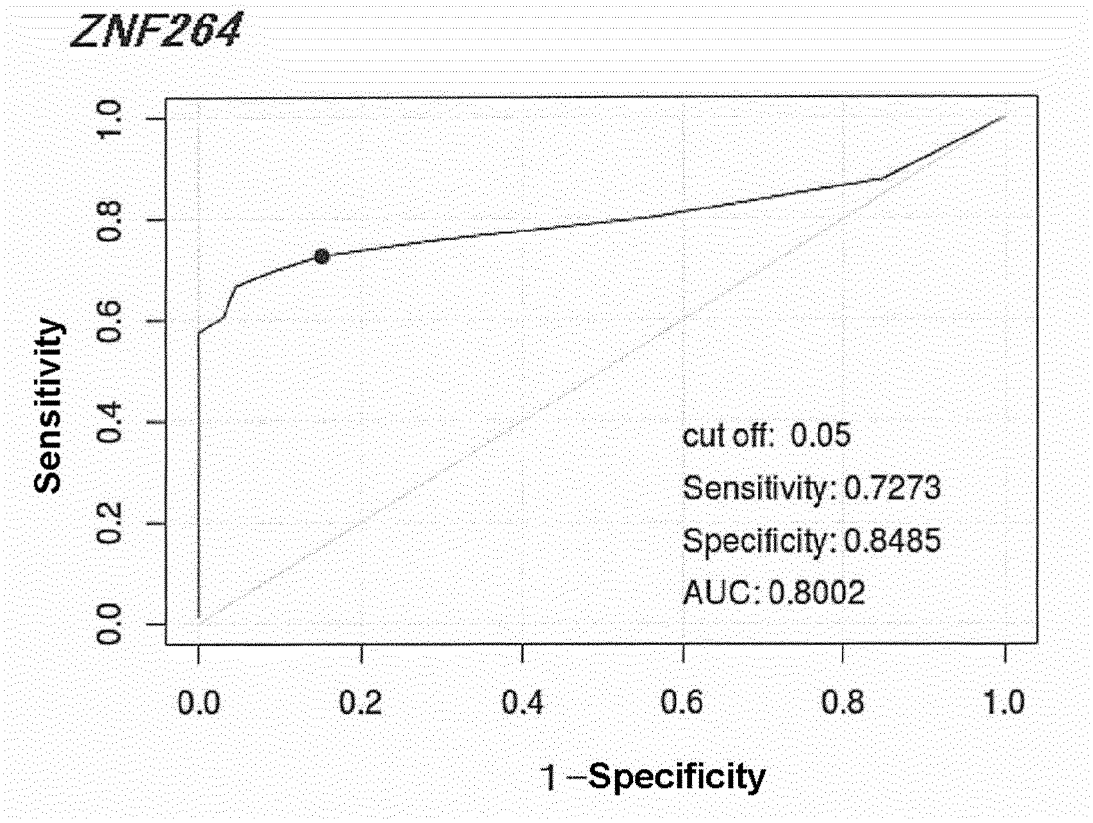


Fig. 12A

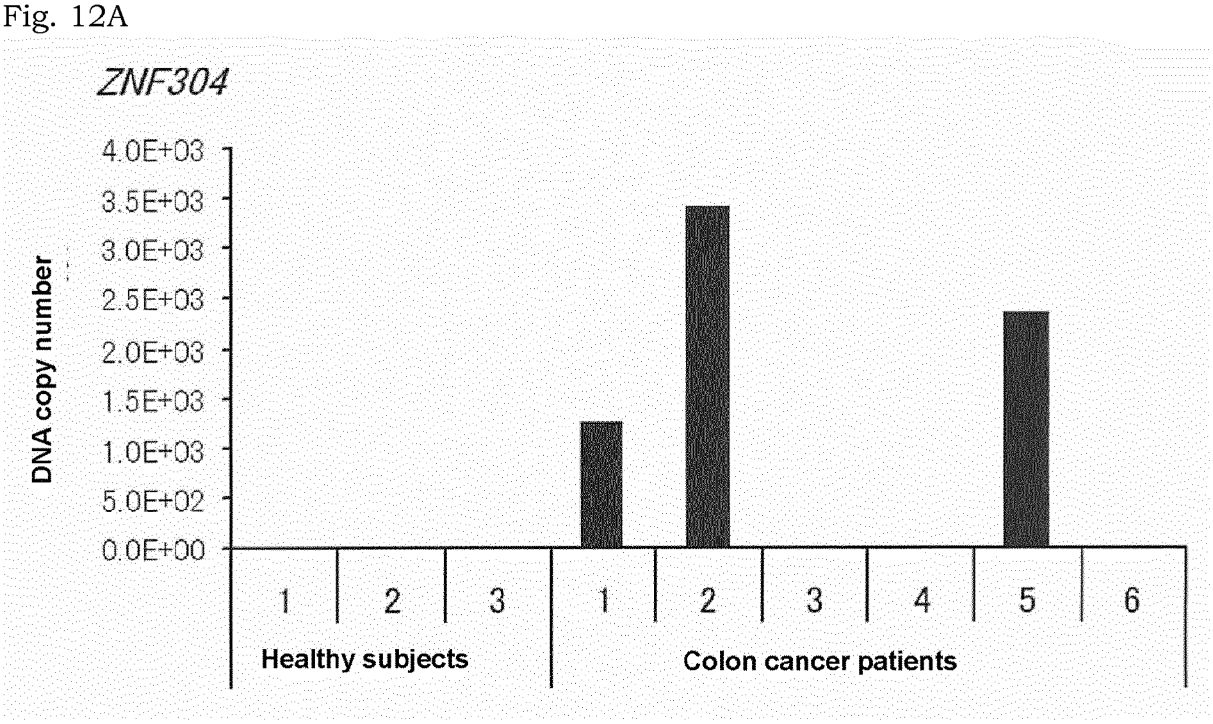

Fig. 12B

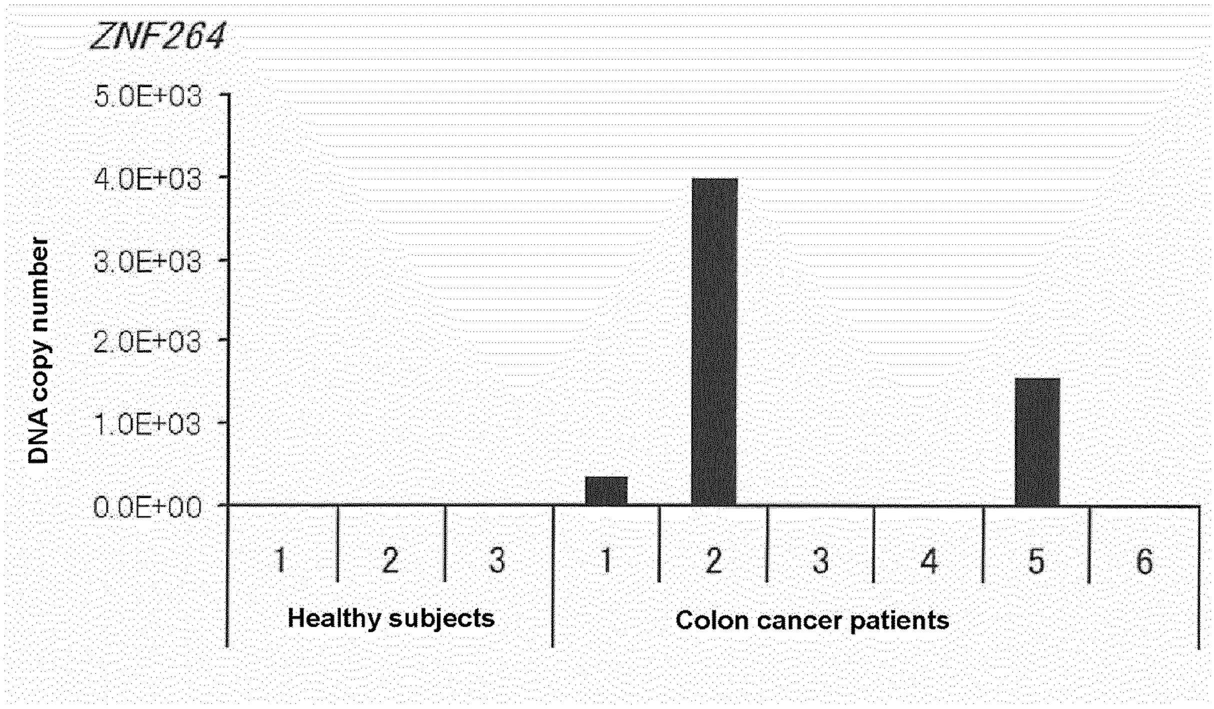


Fig. 12C

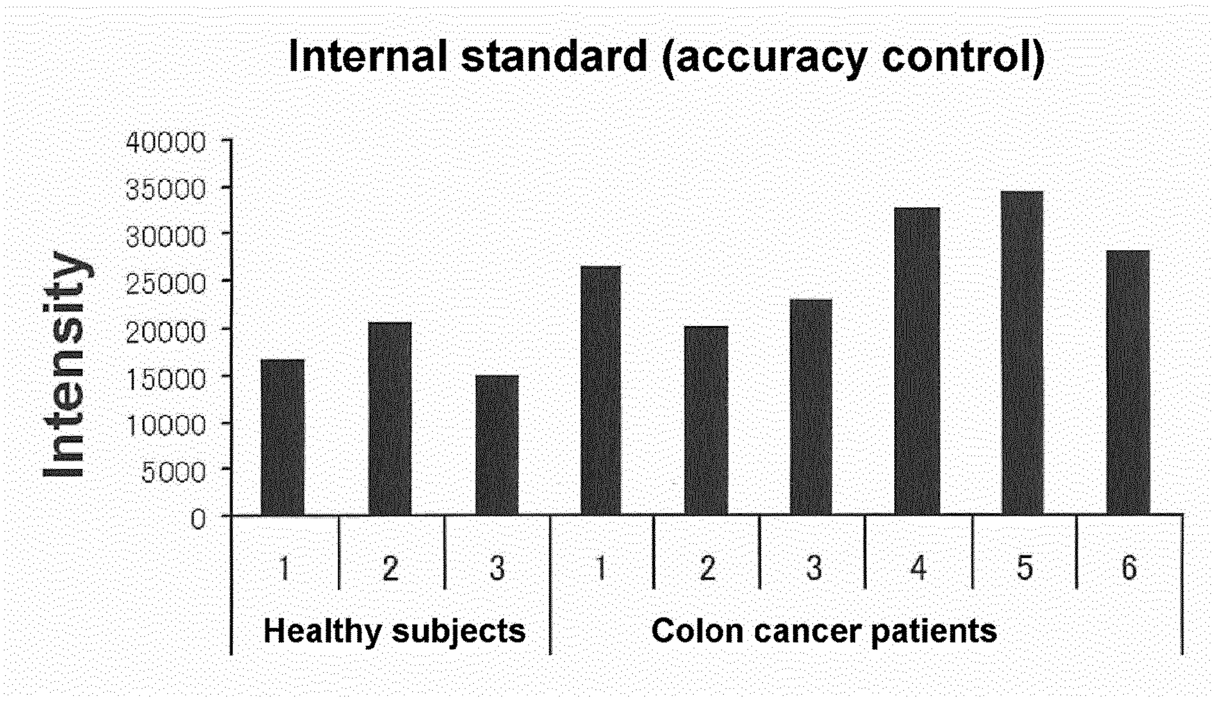

Fig. 13

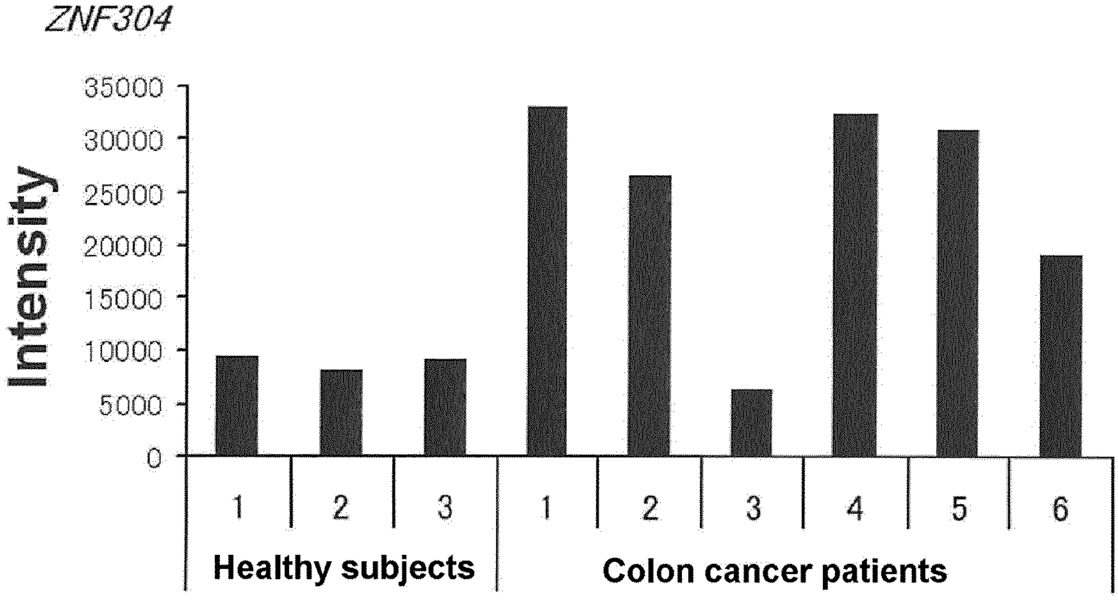
ZNF304
Intensity
35000
30000
25000
20000
15000
10000
5000
0
1
2
3
1
2
3
4
5
6
Healthy subjects
Colon cancer patients

Fig. 14

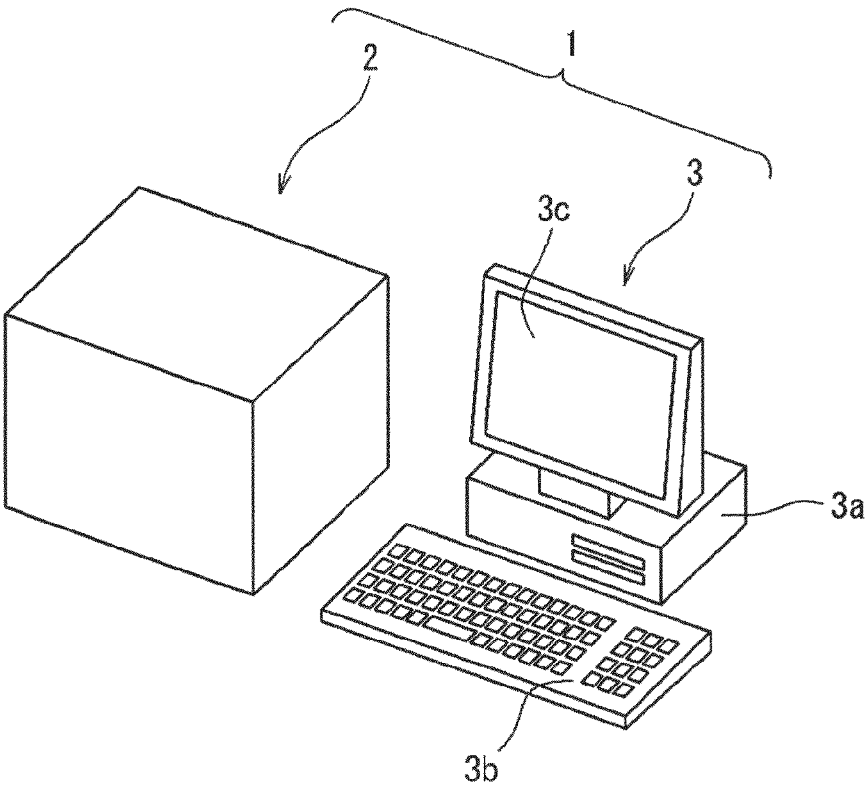
1
2
3
3c
3a
3b

Fig. 15

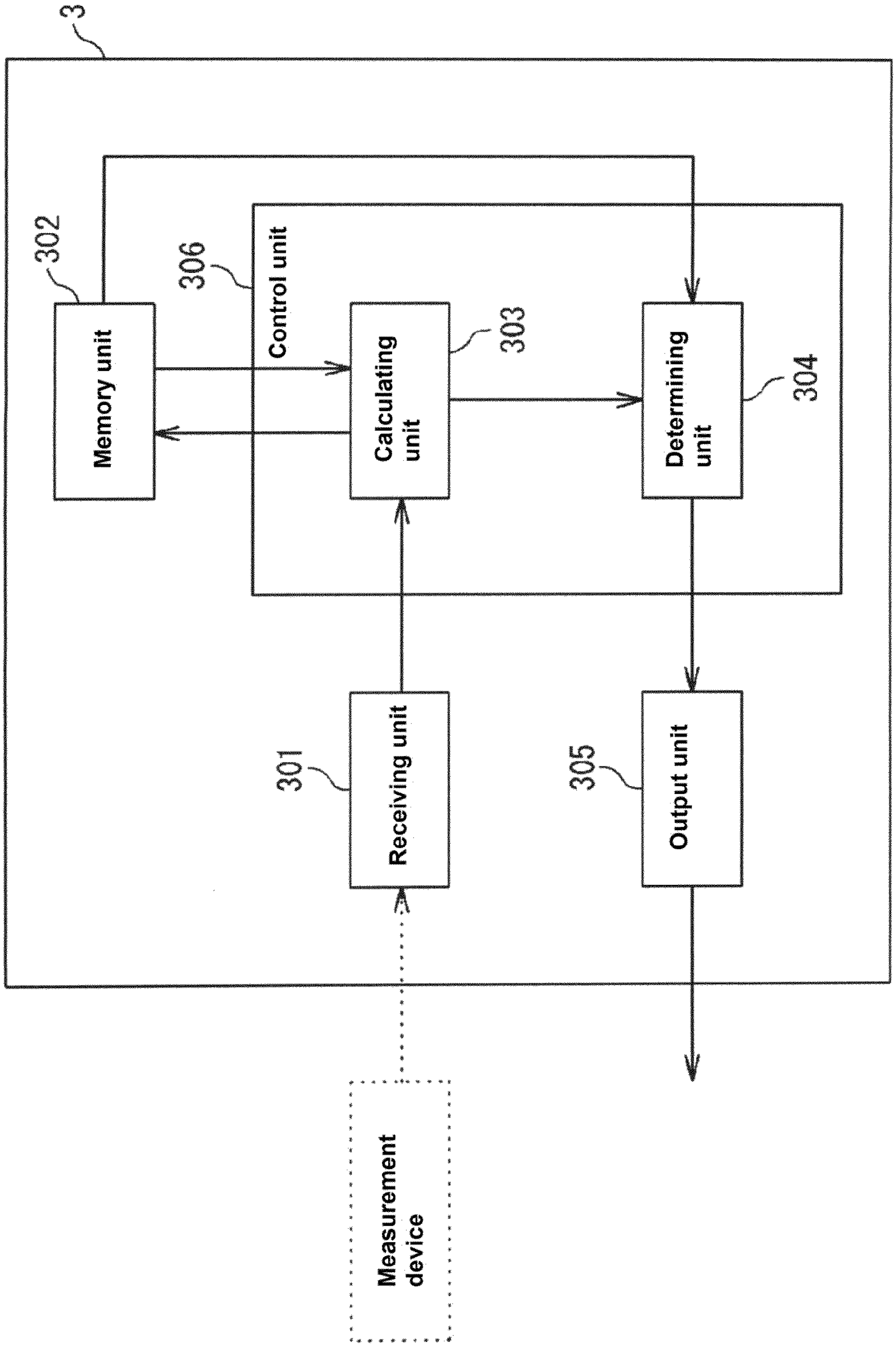
3
302
306
Control unit
Memory unit
Calculating unit
303
Determining unit
304
301
Receiving unit
305
Output unit
Measurement device

Fig. 16

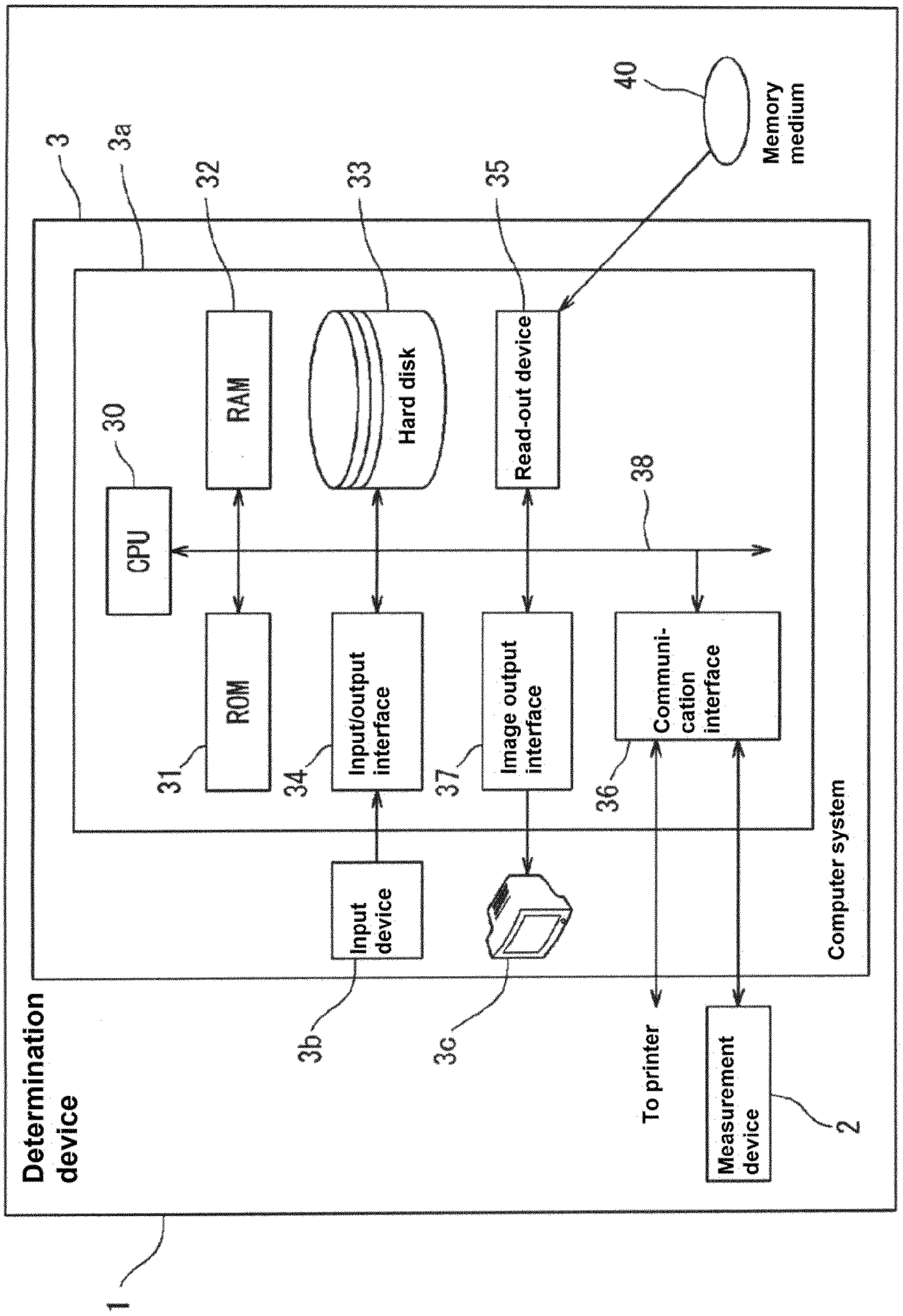
Determination device
Computer system
CPU
ROM
RAM
Hard disk
Read-out device
Input/output interface
Image output interface
Communi-cation interface
Input device
Memory medium
To printer
Measurement device
1
2
3
3a
3b
3c
30
31
32
33
34
35
36
37
38
40

Fig. 17

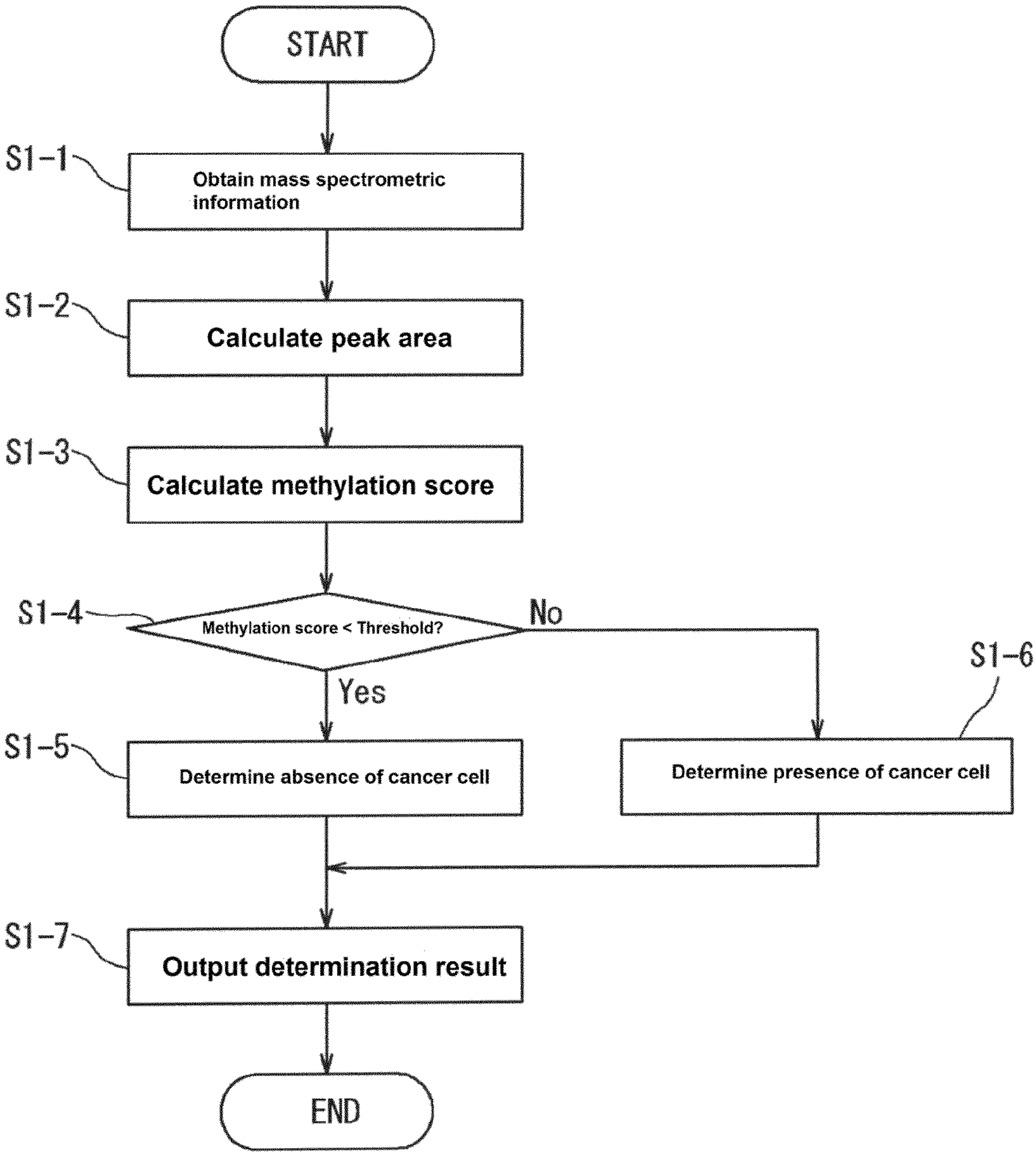
START
S1-1
Obtain mass spectrometric information
S1-2
Calculate peak area
S1-3
Calculate methylation score
S1-4
Methylation score < Threshold?
No
Yes
S1-5
Determine absence of cancer cell
S1-6
Determine presence of cancer cell
S1-7
Output determination result
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2012196756 A
- JP 2012205762 A


### Non-patent literature cited in the description


- **HIBI K. et al.** *Jpn. J. Cancer Res.,* 2002, vol. 93, 883-887
- **QI J. et al.** *World J. Gastroenterol.,* 2006, vol. 12, 7113-7117
- **VILKIN A. et al.** *Cancer.,* 2009, vol. 115, 760-769
- **KIM Y-H. et al.** *Annals of surgical oncology,* 2011, vol. 18 (8), 2338-2347
- **KIM M.S. et al.** *Cancer and metastasis reviews,* 2010, vol. 29 (1), 181-206
- **SELAMAT SA et al.** Genome-scale analysis of DNA methylation in lung adenocarcinoma and integration with mRNA expression. *Genome Res.,* July 2012, vol. 22 (7), 1197-211
- **KOBAYASHI et al.** DNA methylation profiling reveals novel biomarkers and important roles for DNA methyltransferases in prostate cancer. *Genome Res.,* July 2011, vol. 21 (7), 1017-27
- **SALHIA B et al.** DNA methylation analysis determines the high frequency of genic hypomethylation and low frequency of hypermethylation events in plasma cell tumors. *Cancer Res.,* 01 September 2010, vol. 70 (17), 6934-44
- **NAZOR KL et al.** Recurrent variations in DNA methylation in human pluripotent stem cells and their differentiated derivatives. *Cell Stem Cell,* 2012, vol. 10 (5), 620-634
- **REINIUS LE et al.** Differential DNA Methylation in Purified Human Blood Cells: Implications for Cell Lineage and Studies on Disease Susceptibility. *PLoS One,* vol. 7 (7), 41361

SEQUENCE LISTING

<110> SYSMEX CORPORATION
THE UNIVERSITY OF TOKYO

<120> METHOD FOR OBTAINING INFORMATION ON COLORECTAL CANCER, AND MARKER AND KIT FOR OBTAINING INFORMATION ON COLORECTAL CANCER

<130> 12-011JP1

<150> JP2012-205762
<151> 2012-09-19

<160> 23

<170> PatentIn version 3.5

<210> 1
<211> 4176
<212> DNA
<213> Homo sapiens

<400> 1

```
ccactgccgt ccaggtgcct aagcaacaga cccagttttc tctcagtgtc cagtttcaac      60
tttcttactg aataacagca ttaccaccat gatttgaaga taccccatcc cactccacac     120
actggccacc atttttttca acttcttcaa cagaaccctt ccccagaact aaaaacctgt     180
gtgttttcct gaggcctcaa ctaagatgac aactgagacc tcttaaccta aacatgacca     240
aagcctaact cctgatataa ccactaaaaa tcatacctgc tgccaacttc tctatctcag     300
ttgatggcat tttcataatt ttaccagcta cagcaaaaac aaaccaacct tagggtcatc     360
cacgactctc ctctctttca cctttcgcat caaaaaatcc aatcacccta ctttaaaaaa     420
cagaaaattt accaacttct ctcacctcca cagccacacc tctagaccag acaccatcaa     480
cactactgca gtagctgctc cctcatgctc acagtctgtt ctcaacctga aagccagaga     540
gagcatgtta aaatctgagt cagatcatct ccctcctctg ctccaaatct cccacgactt     600
ttaccacact caagacacct cggccagcca ttcctgccta actccttcct ctgatacctg     660
tttccaacaa aggtaattaa gacctgagat tccctgagcg ctccaaactc aatcctacaa     720
gcatttgagc gtacctgttc ctaggtctga tatacttttg cagtactcat cccattggtc     780
gccaggtggg gacttctcca cataactcca tacaactcat ggcctggatt caggacacta     840
aactagaaat gacctccatt cactgccccc tgtctcaagt gaagacagtc atgtgaagaa     900
tcccaggata tcatcactat gtctcatcag agaatctggt cagccttcct tataaaatac     960
atacaaaacc caaaacccat acattctcta acctctgctg ccaccactct ggcccagacc    1020
atcaacgctc acctggacta tttcagtaga cttcatcttg atctccccgt ctaccctata    1080
atccctagtc tacccactct gcagccagag ggagcctttt aagaactgcg tcagaggacc    1140
tcccttctct gtaccaaacc ttgcatgact cccagcaccc tcagaagaga ggaccagctc    1200
```

ctcgggcggc cattccaggc ctacctctga cccccagtc cctgttctca ccaggcacag 1260
agagttcccg gaactcccca aactcattct cctctccatg catgcatttg tacgaactgc 1320
ccctgcgcct tcgagtccgt tccacacctt atgccactga tgaggggagc ggaacccctc 1380
cacataatgc gacggagtga gcaccctgaa gtgcgcgatg agagcgaaca tcccaggaca 1440
caccaaccag gccggggtgc gcttgaggga tgcccccact cacctgaacc cggtccatca 1500
gcaccgccgc tgccatgaga agcggatgaa cagtctgtgc cctgtgggct cagccgaaac 1560
cccacgcctc caccgcgctg gacgccctcg ggctgagtgt agcactccaa gtctcaatct 1620
acccggtgta aaaacgctca caagactaag gcggctgctt ctgggggaga caaggacaag 1680
aggacgtgtg tggaggcctc ggtaagagcc cagccccggc agacacctcc gcgagaggcc 1740
gcccgtccca cgacaggggt catggattgt aacaaaagcg cgaaggaagg tcctgcagtc 1800
ttcactgtct ttctttgcta ggttccacga gagccgcgta gaaagccgac ccgatctcta 1860
gaactcagcc ttcacaacca gaatgcacgg accgggcata ggaaaaatac gtcacgacgg 1920
cgacgccaga agtctcgccc ctgcagccca gagcagcagg aaacgcccag tttctgggct 1980
gtcattggct gcacgccggc taacaatgag ctgggcacag atttctgggt aacgtagttt 2040
gtaccctgca tccaaccagg caacgagcgc ctttcgggtt ctcccggatg tctgccaaga 2100
ggcgtcgtgc agggaggggg agaaaagact gggaaaacga tatctcggag gccacagctg 2160
cttttaaagg gtatgcaccc tgatttccat ggccacctgg gacaatgcca cgccactgaa 2220
gtcgccagca agggacccat tcttcaatcc tgcttctgag gccctgggaa acctgcttca 2280
cctctctcag atttggggat tgaaacgtat taataaattg aacgttgtct gtatttcgtt 2340
tgtgggcctt tttgtaaaac attgaatgtc cttctaattt ttctaacata taaagaaaag 2400
agatgtaatt agctaccttt ctgctggctg tacaggaagc atggtgcccg tatctgcttc 2460
tggtgaggcc acagggaact tttattcatg gcgaaaagtg aaggaggaac ttacatgtaa 2520
catgacgaaa acatgagcct cttctaattc attgcttccc aatttgatat gtagttttac 2580
agcatcagct tcactatgaa cttaaaaaac ctgtgaattc ttggacccaa cctatacctt 2640
ctgataaact ctggggatgg gaccctagct gaagttcaag aactggaggt cttttaaagt 2700
cattagccaa aaacaaaaca aaacaaaaac aaacaaaaaa aaatcagtaa gatgaaaaac 2760
actacaacca acctcattag gaatatggga agtgaggtaa aaagtactta acaccaggcc 2820
tggcacatcg tgctaaatac atacaagctg ttatcagtct tcctcaacac aaggttctca 2880
catacactgc tttaggaaga taaatcaaag atccaccttt tcaggatcag tgtgattttg 2940
aaaaaaaaag aaaaaagtac aattattccc tcactcatca aatagcatca tagataatag 3000
ttataatttt tttttgagac agagtttcac tcttgttgcc caggcttaag tgcaatggca 3060
cgatctcagc tcactgcaac ctctccctcc caggttcaag cgattctcct gcctcggcct 3120

cccgagtagc tgggattaca agcatccgcc accatgcccg gctaattttg tatttttagt 3180

agagatgggg tttctccgtg ttggtcaggc tggtctcgaa ctcctgacct caggtgatcc 3240

aactgcctca gtcttccaaa gtgctgggat tacaggtggg agcccctaat ggttataatt 3300

tttgcacaaa agcacaccaa tatatcaata gactcattta attgaaaaaa atcacatgca 3360

agagggtttc ataaactaca ggtaggtcta tcgtccagaa gacacattct tataggctgt 3420

atgattcaaa acacagaact atcaagacta attgctcttc ataattttta actccactgt 3480

ccacttaaga gtcactgtag ttaaaatttt ataacctggt ttattttatg agatgacata 3540

gaaataattc atatctttga gaaaaaatat atatacacac atacacacag catttatata 3600

tatatacaca tgtagtttat atacacacat acataattat atatatgtat aatataaatt 3660

atctgtctcc actcaaagcc aatataaatt taggtgtcag gaacacatgg aataacatac 3720

ggagtcagtt acagatataa ctgccagcag atttctatgc acgacaaatg cactggctaa 3780

gaaaaactga aataagacta tttgaagccc aaaatgagaa atttaatgga taaaaataac 3840

tggcataaaa taacactttt aagatccttc ttaaggttga aggaaagtga tcccaggtga 3900

tggtctgtga tgatagaaga aatgaggatc tgagagagga gtgcatctat ggattaatga 3960

gaccactcat tatctgtata aaatattaat actgttgggc gcggtggctc acgcctgtaa 4020

tctcagcact ttgggaggcc aaggcaggcg gatcacgagc tcaggagttc gagaccagcc 4080

tgtccaacat ggtgaaaccc tgtttctact aaaaatacaa aaattagcca ggcgtggtgg 4140

tgggcgcctg taatcccagc tactcagaag gctgag 4176

<210> 2
<211> 4220
<212> DNA
<213> Homo sapiens

<400> 2

agaggccttc ttgtgcagcc aggcctggag tcagaagatg gaatgaagga agtggcaggt 60

cacctgcatg gtggaggggg aaggaaggcc ttacccagag acaccaggag cccacagttt 120

tccagcatca cctcctggta cagggtccgc tgagctaggt ccagctgccc ccactcctcc 180

ttggtgaaag tcacagccac atcatcaaag gtcacagaca cctggaaagt caaacagaat 240

tagtagtatt ggcctttcag ctgctgaatg agatcacaga ctgtgctcca ctggaaaagg 300

tgtacagtga tggcggcctg aatcacagag cacctcctga gggcctagct ctgtgctggg 360

ctcaagaggg aagatagatg cttttaacat aataccagag taaaggagag aagcctccta 420

tttaacggcc gttagagaat gacacaggac aacggaaaca acttgtggac tgtgaaatac 480

agaagctaag ggctctaaaa cgacagggaa atggggaaat ggtaataata gcaataatgg 540

ttacaacggc agtacctgag tctcactggt ccagctaggt gcctgcccca tgttgaaggc 600

ttcacatcct gtgtaaacac agtgactaga agttcctagc tccaaacact tgaaactgac 660
acttgacagc tcttacctca aagacagcat ttcatttctc tgagccttga tttctggatc 720
aatacaataa cacttcccac cagtaacagt tgttaaattt aaagtattca atgctcagtg 780
tgctctggga tttttattaa tcctttcaca gtccctgcag tcaacaccat catcaccttc 840
acagtcaatg acttcatgct aaggaagcgc tatcaattca cccagtcaca catctcacca 900
ccatagatag gatctaaaca tggggttact atgattcacg gtctaacagt gtaactccaa 960
cactccccca acatcaaagc aaatctaatg cgatgaagtt ataagcattt ccactcattt 1020
ctaacattaa gtctatttca attagccctg aaaaacgtat ctgtgggccg ggcatggtgg 1080
ctcatgcatg taatcccagc actttgggag gtcgaggtgg gcggatcgct tgaggccagg 1140
agttgaagac cagcctggca aatatggtga aacccagtct ctactaaaat acaaaaatca 1200
gtcaggtgtg gtagtgcatg cctgtaatcc caactactcg gcaggctggg gcaggagaat 1260
tgcttgaacc tgggaggcag agactgcagt gagctgagag tgcaccactg tactccagcc 1320
tgggcgacag agtgagaccc tgtctcaaaa caaaaaaaag tatgtgaaca cgggcacaaa 1380
aagctcagca ttcctcactg accccacaga gcggcactgg aagggctgca tctacttggg 1440
gaacgacagg ctccagacca gcttcccatg ccctctggga tctccctggg tctcccagat 1500
cccccaagga aattccacaa tgaaccaacc atcggcctct ttccttactg ggttccgaca 1560
gaacttgtct ctgacgccag gatactttgg ggccacggaa cacgagcggc caaggataat 1620
gcaggctgta gcgatgagag taagggcatc tgactggcca caatgtctgc agtagttgcg 1680
gatggctaga acatccagga aaagaccaca cactgttgcc ctccacgtct cagcggccgc 1740
aggaaaacca tggcaacctt acacaccctc ctgagcctcc ttaaacatac aacatacctt 1800
gtctctgctc gcgcatttgt cggcttgcta ctgccctgcc ctccccttcc cccctcaggc 1860
ccccaattca tgctggaatg agacctggtc caccagagaa tgcttgcagg ccaaggatga 1920
tgggagtccc cagaggaagc atcattcact caacaaccta cggagctcct tccagtaggc 1980
ccaacactcc ttacggccag gtataaatta agtggcaaac cagctccaca caccaaagcg 2040
aacgacgact gcgaagacaa atccagggga tacctgggct cccacccact tcggaaaccg 2100
cctcagcgct ggcaaggaag cggcaaccgc gaagccaccg tccactcacc tgggcccggt 2160
ccgtcagcac cgctgccgcc atcacgtcac cccctgggcc gcagagctgg gacagaggag 2220
ccccgggcgg cctgacggac cccacctgtg cccgaccctg gccggttgcc cggggcttcc 2280
aggcgcgctc ggggccgccg cctcctcggt ggcaacggcg ttccagacgc agggccgccg 2340
cctcctcggt ggcaaccgcg ttccagaccc agggcgccgc ccctggagac cgcggggctg 2400
aagggcgggg gaggagagac cgcagcttga cggcgaccca ggaacaaacc cacggaaggg 2460

gtcaggctcg tacaaaggtg cggcggggat tcctatccta ctcccctgct cggtcctgac 2520

tagggacctg caaagcggcc ccgaccgggg ccctcagtgg ctacagacct ctccagacag 2580

aagtgcagaa gaaaatggcg tccgcagcga gcaacccgga agtcccgccc ccaccggcgt 2640

gagccgtcgg aaacgtcccc gtcctaggcc gtcgttggcc cagctcccgc cgtacgcata 2700

atgcacagcc ttctaagtag tgtagttttc ctcatgtgtt caacaacggg aggaacgctt 2760

cacggcgcaa gttttcctcc cagggaaagg cactccagga cccgaggaga cgctccagac 2820

ccacctcttt acggtgggca caactcgagg ggaaggatgg agggagggaa ccgaggggca 2880

agggttacat tgcattcttc tctcgggaaa atcggagctc gggagacagg gactttcctt 2940

caggtcaggg cacaggcaaa ggcgcagaac tgggtcctca gctcgcctgg cgactgaccc 3000

cctttcacat ccctcctccc cggatcatca tcttggaaga cacaaaaaag ccgaatgatc 3060

atctggaagc cctgccccga cacgttgtgt gcgcaaactg ccctcttttg agccttcttt 3120

ggaccagcac agcctctacg tgatgcaggg agtcatgtat ttggcccaga ccacacaaaa 3180

agtggatttg cccacttctg aggtggggag gctgcaagtg gaggaggttc ggaggctcag 3240

gatggttagt ttgaaatgtc tacagaacca caaagtatct ctgtcacagg caattcgact 3300

tacgactgaa tgttggaaga aggtttgagc atgaatatat gaatttagga gttaccttac 3360

actgttattt caagtcatga tattgagtga gaccatcaag gcaatgcatg tagatggaga 3420

agaaaataca cagagcatga gccggggacc aagtctacat cattaaatgg ttgatgacaa 3480

tattaccaga aaggccaggt ccttgcatct agatcctgct gcttgctgca cagaaggcca 3540

gtcagcaaga caatgagtat tgtcagggat gaaggcttta ttcggtgctg cagctgagga 3600

aaatgggaga tcaatctcaa atctctttcc tcaactgact aaaactaggg gtttatacat 3660

cagggaagaa gtgtaactat gtttgagaaa acaggaatta ggaaggggta aggaagagga 3720

atgggtcaac aggcagcaga tggtcagtta ggcaatcatc atgggtaagg ggtatagcat 3780

ctcgtttttc cagatgtggt aatccagtaa gtttcagttt ctttatacta tctgggaggc 3840

ctgatgcttg gtttcctgag aaaggaaatc agataagaca aataaaagtg tctcgagttt 3900

taggaagggg aggctcaatg tctataatgt gttgattcaa aagaaaccat aaatatcagt 3960

tctatgggac actggagctg gtttcagttc cctgtttcta tttatcaatt cctcaatcat 4020

ggggaatctg gacgtgatct ttctggctgc tttatgctga ggaggggtat tgtgggtggc 4080

tgcataccat agatgaccac gtggccactc agattaactc aaaggttaat ctaacgctat 4140

agttttcttt ctttctttct tttttttttt tgagacagag ttttgttctt tcacctaggc 4200

tggagtgcag tggcacgatc 4220

<210> 3

<211> 21
<212> DNA
<213> Artificial Sequence

<220>
<223> primer

<400> 3
taataatgag ttgggtatag a 21

<210> 4
<211> 20
<212> DNA
<213> Artificial Sequence

<220>
<223> primer

<400> 4
acataccctt taaaaacaac 20

<210> 5
<211> 21
<212> DNA
<213> Artificial Sequence

<220>
<223> primer

<400> 5
gtataaatta agtggtaaat t 21

<210> 6
<211> 20
<212> DNA
<213> Artificial Sequence

<220>
<223> primer

<400> 6
ctcctctatc ccaactctac 20

<210> 7
<211> 10
<212> DNA
<213> Artificial Sequence

<220>
<223> Tag sequence

<400> 7
aggaagagag 10

<210> 8
<211> 31
<212> DNA
<213> Artificial Sequence

<220>
<223> T7 promoter sequence

<400> 8
cagtaatacg actcactata gggagaaggc t 31

<210> 9
<211> 25
<212> DNA
<213> Artificial Sequence

<220>
<223> primer

<400> 9
ttgtatttaa ttaggtaacg agcgt 25

<210> 10
<211> 25
<212> DNA
<213> Artificial Sequence

<220>
<223> primer

<400> 10
tttaaaaaca actataacct ccgaa 25

<210> 11
<211> 24
<212> DNA
<213> Artificial Sequence

<220>
<223> primer

<400> 11
atttatttcg gaaatcgttt tagc 24

<210> 12
<211> 25
<212> DNA
<213> Artificial Sequence

<220>
<223> primer

<400> 12
actctacgac ccaaaaaata acgta 25

<210> 13
<211> 176
<212> DNA
<213> Homo sapiens

<400> 13

taacaatgag ctgggcacag atttctgggt aacgtagttt gtaccctgca tccaaccagg 60

caacgagcgc ctttcgggtt ctcccggatg tctgccaaga ggcgtcgtgc agggaggggg 120

agaaaagact gggaaaacga tatctcggag gccacagctg cttttaaagg gtatgc 176

<210> 14
<211> 220
<212> DNA
<213> Homo sapiens

<400> 14

gtataaatta agtggcaaac cagctccaca caccaaagcg aacgacgact gcgaagacaa 60

atccagggga tacctgggct cccacccact tcggaaaccg cctcagcgct ggcaaggaag 120

cggcaaccgc gaagccaccg tccactcacc tgggcccggt ccgtcagcac cgctgccgcc 180

atcacgtcac cccctgggcc gcagagctgg gacagaggag 220

<210> 15
<211> 33
<212> DNA
<213> Artificial Sequence

<220>
<223> primer

<400> 15
gggatattaa gtggagttat tttggtttta gtt 33

<210> 16
<211> 21
<212> DNA
<213> Artificial Sequence

<220>
<223> primer

<400> 16
ccctcccaac atccttccta a 21

<210> 17
<211> 123
<212> DNA
<213> Homo sapiens

<400> 17

ctgcatccaa ccaggcaacg agcgcctttc gggttctccc ggatgtctgc caagaggcgt 60

cgtgcaggga gggggagaaa agactgggaa aacgatatct cggaggccac agctgctttt 120

aaa 123

<210> 18
<211> 124
<212> DNA
<213> Homo sapiens

<400> 18

**acccacttcg gaaaccgcct cagcgctggc aaggaagcgg caaccgcgaa gccaccgtcc 60**

**actcacctgg gcccggtccg tcagcaccgc tgccgccatc acgtcacccc ctgggccgca 120**

**gagc 124**

<210> 19
<211> 23
<212> DNA
<213> Artificial Sequence

<220>
<223> primer

<400> 19
taatgatagt ttagaaattg ggc 23

<210> 20
<211> 23
<212> DNA
<213> Artificial Sequence

<220>
<223> primer

<400> 20
gaacataaaa aaaatacgtc acg 23

<210> 21
<211> 33
<212> DNA
<213> Artificial Sequence

<220>
<223> primer

<400> 21
gggatattaa gtggagttat tttggtttta gtt 33

<210> 22
<211> 21
<212> DNA
<213> Artificial Sequence

<220>
<223> primer

<400> 22
ccctcccaac atccttccta a 21

<210> 23

<211> 94
<212> DNA
<213> Homo sapiens

<400> 23

```
caatgacagc ccagaaactg ggcgtttcct gctgctctgg gctgcagggg cgagacttct      60

ggcgtcgccg tcgtgacgta tttttcctat gccc                                   94
```